(19) Europäisches Patentamt
European Patent Office
Office européen des brevets

(11) EP 1 251 843 B1

(12) **EUROPEAN PATENT SPECIFICATION**

(45) Date of publication and mention of the grant of the patent:
**28.12.2005 Bulletin 2005/52**

(21) Application number: **01901275.6**

(22) Date of filing: **19.01.2001**

(51) Int Cl.[7]: **A61K 31/19**, A61K 31/4168, A61K 31/569, A61K 31/496, A61K 31/451, A61K 31/4545, A61K 31/4174, A61K 31/03, A61K 31/58, A61K 31/245, A61K 31/375, A61K 31/4164, A61K 31/167, A61K 31/00, A61K 9/22, A61K 9/20

(86) International application number:
**PCT/GB2001/000201**

(87) International publication number:
**WO 2001/052833 (26.07.2001 Gazette 2001/30)**

(54) **COMPOSITIONS FOR DELIVERY OF A CORTISOL ANTAGONIST**

ZUSAMMENSETZUNGEN ZUR ABGABE VON CORTISOLANTAGONISTEN

COMPOSITIONS PERMETTANT LA LIBERATION D'ANTAGONISTE DE CORTISOL

(84) Designated Contracting States:
**AT BE CH CY DE DK ES FI FR GB GR IE IT LI LU MC NL PT SE TR**
Designated Extension States:
**AL LT LV MK RO SI**

(30) Priority: **21.01.2000 GB 0001449**

(43) Date of publication of application:
**30.10.2002 Bulletin 2002/44**

(73) Proprietor: **Cortendo AB**
**421 30 Västra Frölunda (SE)**

(72) Inventors:
- **MÄRIN, Per**
  **S-426 77 Västra Frölunda (SE)**
- **LANDH, Tomas**
  **S-226 49 Lund (SE)**
- **ÖSTHOLM, Ivan**
  **S-411 34 Göteborg (SE)**

(74) Representative: **Gardner, Rebecca Katherine**
**Frank B. Dehn & Co.**
**179 Queen Victoria Street**
**London EC4V 4EL (GB)**

(56) References cited:

**EP-A- 0 249 587**
**EP-A- 0 872 229**
**WO-A-01/01987**
**WO-A-91/02518**
**WO-A-98/06387**
**US-A- 5 788 980**

- **DATABASE WPI Section Ch, Week 198720 Derwent Publications Ltd., London, GB; Class A96, AN 1987-139154 XP002165358 & JP 62 077335 A (FUJISAWA PHARM CO LTD), 9 April 1987 (1987-04-09)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 251 843 B1

## Description

The present invention relates to compositions and formulations which contain one or more cortisol antagonists, such as a cortisol synthesis inhibitor, methods for preparing such formulations and their use in therapy. More particularly, the invention relates to the use of such compositions in the prevention or treatment of metabolic syndrome and non-insulin-dependent diabetes mellitus (NIDDM), referred to herein as diabetes mellitus type II.

often a person with abnormal glucose tolerance, insulin resistance or diabetes mellitus type II will also have one or more risk factor for cardiovascular disease, stroke or other arteriosclerotic disease such as obesity, especially abdominal obesity, hypertension, dyelipidemia and microalbuminuria. This clustering has been labelled variously as metabolic syndrome, Syndrome X or Insulin Resistance Syndrome. An attempt to more adequately describe metabolic syndrome was recently done (Diabet. Med. 1998, 15, 539-553) and a working definition for the clinical recognition of the condition was suggested to include the following characteristics: glucose intolerance, abnormal glucose tolerance or diabetes mellitus and/or insulin resistance together with two or more of the components: (1) Impaired glucose regulation or diabetes (2) Insulin resistance (3) Raised arterial blood pressure (4) Raised plasma triglycerides and/or low HDL-cholesterol (5) Central obesity and/or a body mass index of more than 30 kg/$m^2$ (6) Microalbuminuria.

Those patients with metabolic syndrome are at very high risk of developing diabetes and therefore vigorous early management of metabolic syndrome may have a significant impact on the prevention of diabetes. All of the physiological imbalances associated with metabolic syndrome are risk factors for cardiovascular disease and thus effective early management of metabolic syndrome may also have a significant impact on the prevention of cardiovascular disease, stroke and other arteriosclerotic diseases.

Commercially available medical treatment for metabolic syndrome is generally lacking. Currently, most widely used therapies include diet and exercise (weight reduction therapy). Other therapies that have been suggested for the treatment are disclosed in US 5,756,513, US 5,849,740, and WO 98/36770, using a dopamine agonist, ketoconazole, or a combination of a cortisol synthesis inhibitor (such as ketoconazole) and human growth hormone, respectively. The use of ketoconazole as described in US 5,849,740 is based on an already marketed oral immediate release dosage form, Nizoral® (Fungoral®), originally developed by Janssen Pharmaceutics (Belgium) for the treatment of fungal infection.

Thus, the utility of a cortisol synthesis inhibitor in the treatment of metabolic syndrome and related conditions has been shown. However, the normal 24 hour plasma profile of cortisol shows a circadian variation with a shallow minimum during the day time (with the exception of short peaks of increased secretion) until around midnight followed by a rather steep increase to a broad maximum during the night, followed by a sharp fall during early morning lasting until around eight in the morning. The present inventors have realised that administration of an immediate release dosage form of active agent, even around 10p.m. as proposed in US 5,849,740, may not provide an effective enough counter to the rising plasma cortisol levels which occur in the early hours of the morning.

Metabolic syndrome is characterised by a breakdown in the negative feedback mechanism which operates along the hypothalamic-pituitary-adrenal gland axis and results in increased cortisol secretion. It is therefore desirable to reduce cortisol activity in the body through exogenous means which can go some way to mimicking regulation by negative feedback observed in healthy people.

Metabolic syndrome and diabetes mellitus type II are typical 'Western' diseases and effective treatment would benefit a very large number of people. Many sufferers are sophisticated health care users and a therapeutic method which was both effective and convenient to administer and receive would represent a significant advantage. A single daily dose is preferred and this should preferably be given at a time of day when the patient is normally awake. Such a requirement is in conflict with the body's normal circadian rhythm for the synthesis of cortisol which suggests that a pharmaceutical such as Nizoral® would best be administered between midnight and three O'clock in the morning. Thus, while conceptually the use of a cortisol synthesis inhibitor (e.g. ketoconazole) in the treatment of metabolic syndrome and diabetes mellitus type II is a significant advance, the therapeutic methods described, inter alia, in US 5,849,740 could be improved both in terms of therapeutic efficacy and convenience of administration.

There have been limited studies on ways of modifying absorption of cortisol antagonists and ketoconazole in particular in the gastro-intestinal tract (Daneshmend, Clin. Pharmacokinetics (1988) 14, 13-34). In particular, attempts have been made to enhance ketoconazole absorption by incorporating acidic components into the formulation but with limited success. Ketoconazole has been administered as a solution or in suspension in an attempt to enhance the bioavailability and/or reduce variability of absorption. A controlled release formulation however has not been proposed.

The present invention addresses these problems. Thus, in one aspect, the present invention provides a composition for controlled release of a cortisol antagonist comprising at least one release rate controlling substance together with said cortisol antagonist, wherein at least one of the release rate controlling substances is a fatty acid ester wherein the fatty acid component is a saturated or unsaturated fatty acid having between 6 and 26 carbon atoms and the composition is adapted for oral administration to a mammal.

By the term 'cortisol antagonist' is meant any compound or agent which reduces production of cortisol or

circulating levels of biologically active cortisol or which limits the biological effects of cortisol by inhibiting cortisol (glucocorticoid) receptors competitively or non-competitively, or in any other way which interferes with the regulation of cortisol synthesis along the so-called hypothalamic-pituitary-adrenal gland axis. Thus a "cortisol antagonist" may broadly be regarded as any compound or agent which antagonises or inhibits (i.e. reduces or prevents) cortisol activity.

A large number of agents are known to suppress glucocorticoid production or inhibit their receptor binding in humans: sodium valporate (Aggernaes, H. et al. Acta Psychiatr. Scand. (1988) 77 170-174); Enkephalins and their synthetic analogues (Stubbs, W.A. et al. The Lancet (1978) 1225-1227); Opioids such as loperamide, commercially available under the trademark IMODIUM from Janssen Pharmaceutica N.V.; the antihypertensive drug Clonidine (Slowinska-Srzednicka, J. et al. European Journal of Clinical Pharmacology (1988) 35 115-121); Oxytocin (Legros, J. J. et al. Endocrinologica (1987) 114 345-349) and Mifepristone, known as RU 486 or RU 38486 available from Roussel-Uclaf .

Any of the above agents or any of the large number of cortisol synthesis inhibitors known in the art, e.g. those containing an azole group such as econazole (Squibb, U.K.), ketoconazole and miconazole (Janssen, Belgium) and their derivatives, may be used as cortisol antagonists according to the present invention. In the case of econazole and miconazole, their derivatives being preferred.

Other agents known to have effects on cortisol secretion and/or activity include aminoglutethimide (Elipten®), metyrapone (Metopirone®), etomidate, trilostane, mitotane (Lysodren®) and Trilostan. Phenyltoin (dilantin, diphenylhydantoin, DPH), procaine, vitamin C, salicylates including Aspirin, cimetidine and lidocaine (KRONOS) are further pharmaceuticals for which a cortisol antagonistic activity has been observed (Med. Hypothesis 13 31-44, 1984). Also, other imidazole derivatives than ketoconazole and its derivatives are included in the invention. Furthermore, certain phosphatidylcholines and serines are currently promoted as cortisol synthesis inhibitors for the treatment of increased cortisol secretion induced by the stress caused by too much exercise.

Further cortisol antagonists, particularly melengesterol acetate and its derivatives are described in US 5,252,564.

Preferred cortisol antagonists include those compounds which inhibit the synthesis of cortisol, either by reducing the production of cortisol in any form or which cause the production of a modified form of cortisol which is less biologically active than native, naturally occurring cortisol. Preferably, cortisol synthesis inhibitors will act on the cortisol synthetic pathway in a way which does not significantly affect the normal production of the other steroid hormones. Ketoconazole and its derivatives are preferred for use according to the invention (some derivatives of ketoconazole are shown by formula (I) herein) and in addition, isomers of ketoconazole are known and may be used, individually or in combination (Rotstein et al., J. Med. Chem. (1992) 35, 2818-2825). The Cis-2S,4R and Cis-2R,4S isomers are particularly preferred for use in accordance with the present invention.

The composition and the cortisol antagonist it contains may be in any of the following forms (or mixtures thereof) immediately prior to administration to a subject: solid, solid solution, semi-solid, solid dispersion, amorphous (liquid or solid), glassy, liquid crystalline, liquid matrix, or liquid. The cortisol antagonist is typically dispersed in a release rate controlling substance which is a solid, semi-solid, amorphous, liquid crystalline or liquid matrix. The cortisol antagonist may be dissolved, dispersed, emulsified or suspended in the afore-mentioned release rate controlling substance.

Ketoconazole is a particularly preferred cortisol synthesis inhibitor for use in the treatment of diseases characterised by or associated with hypercortisolemia such as metabolic syndrome and diabetes mellitus type II. However this compound is difficult to formulate and administer, in particular due to its low solubility. It is therefore surprising that effective controlled release compositions including ketoconazole as active ingredient can be prepared.

It is particularly surprising that in the compositions of the present invention, the active ingredient can be administered in solid or semi-solid form and this comprises a preferred embodiment. Thus in a preferred aspect the present invention provides a composition for controlled release of a cortisol antagonist, wherein said composition is adapted for oral administration, comprising at least one release rate controlling substance, wherein at least one of the release rate controlling substances is a fatty acid ester wherein the fatty acid component is a saturated or unsaturated fatty acid having between 6 and 26 carbon atoms, and a pharmaceutically effective amount of a cortisol antagonist, said cortisol antagonist being in solid form.

By 'solid form' is meant that the cortisol antagonist in the state it is in immediately prior to administration to a subject is not in solution, i.e. not solved or solvated or significantly, e.g. less than 40%, preferably less than 20%, most preferably less than 10% soluble in the composition or any of the components of the composition and mixtures thereof. The cortisol antagonist is thus typically in solid, crystalline or amorphous form and the composition as a whole is preferably a solid to semi-solid, essentially water-free system (e.g. less than 10 or 5% water) in which the cortisol antagonist, which may conveniently be micronised (or nanonised) and/or crystalline and/or amorphous (optionally micronised), is dispersed. For the purpose of the above definitions, the temperature of the composition and its components immediately prior to administration is considered to be room temperature (i.e. 15 to 30°C).

However, the compositions of the invention need not be in solid form immediately prior to administration but

may exist in liquid form or as an emulsion. In this case, suitable active agents include those being soluble in, e.g. microemulsions, polymeric micelles or mixed micelles of lipid-polymer mixtures and surface release rate controlling substances may include A-B-A type of block copolymers such as poloxamers, in particular Pluronic F68 and Pluronic F127. Pluronic F68 and F127 are characterised by a heat induced transition from liquid to semisolid (Drug release from Pluronic F127 gels, Chen-Chow, Pai-Chie, PhD Thesis, The Ohio State University, 1979). Also polar lipids exhibiting a phase behaviour characterised in that they show a liquid to semisolid phase transformation. Particularly useful monoglycerides are glyceryl monooleate and glyceryl monolinoleate which exhibit a transition between liquid reverse micellar phase to semisolid cubic liquid crystalline phase.

While ketoconazole, including its isomers, is particularly preferred for use in the compositions of the invention, further preferred cortisol antagonists for use in the compositions of the invention include derivatives of ketoconazole. Such compounds include structurally related compounds which have a similar ability to reduce cortisol activity in the body. Such derivatives having at least 30%, preferably at least 50% or at least 75 or 85% or more of the anti-cortisol activity of ketoconazole itself. Chemical modifications to ketoconazole which may be made without significantly reducing the activity of the compound will be obvious to the skilled man and/or may be prepared and tested by routine experimentation or modelling. At least the group of compounds represent by formula I below can be considered as derivatives of ketoconazole.

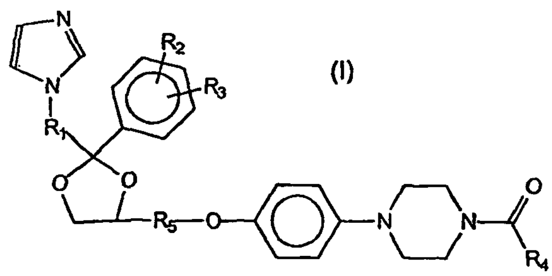

(I)

wherein $R_1$ and $R_5$ which may be the same or different represent a $C_{1-3}$ alkyl group, $R_2$ and $R_3$ which may be the same or different represent hydrogen, a halogen atom or a methyl group substituted by one or more halogen atoms and $R_4$ represents the group $(CH_2)_nCH_3$ wherein n may be 0, 1 or 2. Ketoconazole itself is a compound of formula (I) wherein $R_1$ and $R_5$ are $CH_2$, $R_2$ and $R_3$ are Cl and $R_4$ is $CH_3$.

Throughout the specification, the term "ketoconazole" is used to refer to ketoconazole itself in all its isomeric forms and derivatives as discussed above.

By 'controlled release' is meant that the active ingredient is released from the formulation and thus made available for uptake by the body in a regulated manner, this being determined by the release rate controlling substance and interactions between the active ingredient, the release rate controlling substance and the media surrounding the formulation (e.g gastric juice). The compositions of the invention will conveniently be time-specific controlled-release systems, i.e. formulations that are designed to release the active agents in a specific temporal pattern. Release of the majority of active ingredient is not immediate and may, for example, be delayed, sustained or extended. Thus 'controlled release' includes sustained, delayed, extended or modified release, as understood in the art and as may be contrasted with immediate release dosage forms.

The term 'controlled release' is well known in the art and typically relies on a change of state within the formulation after administration which results in release of the active agent from the formulation. The change of state may be caused for example by changes in the temperature, pH, ion concentration etc. in the environment surrounding the composition. The change of state may involve a phase transition and/or gelling.

The compositions of the present invention provide a very effective and flexible way of controlling the release of the cortisol antagonist. In a preferred embodiment of the present invention, the in vivo uptake of cortisol antagonist is sustained for at least 0.5 hours, preferably at least 1 or 2 hours, more preferably at least 3 or 4 hours, particularly at least 6 or 8 hours. In the context of the treatment of Metabolic Syndrome, release will preferably be sustained for 1 to 12 hours, preferably 2 to 12 hours, more preferably 3 to 8 hours e.g. between 4 and 6 hours.

Sustained release will result in a pharmaceutically effective amount of cortisol antagonist being available for absorption and preferably in the plasma of the patient for the time periods discussed above. In the context of the present invention, a pharmaceutically effective amount (level) will be one which is able to cause a physiologically significant reduction in cortisol activity, typically a physiologically significant reduction in circulating levels of cortisol. Levels of cortisol antagonist which are pharmaceutically, i.e. physiologically effective and reductions in circulating levels of cortisol which are physiologically significant will vary from patient to patient but can be readily determined, particularly with reference to the parameters of interest in the conditions being treated. By way of example, for ketoconazole,

sustained release will preferably result in circulating plasma levels of ketoconazole in excess of 100 ng/ml (typically in excess of 200, 500 or even 1000 ng/ml) for at least 2 hours, preferably 3 or more, more preferably 4 or more, e.g. 4-8 or 4-6 hours.

It will be appreciated that circulating plasma levels of a cortisol antagonist which are physiologically effective will not only vary from patient to patient but from one cortisol antagonist to another. For the avoidance of doubt, if the levels of cortisol antagonist are not uniform in the plasma of the body, it is the circulating plasma level in the relevant organ, e.g. in the adrenal gland which is of most importance.

The *in vivo* release rates of active ingredient from the formulations of the invention can be correlated with the *in vitro* dissolution rates of the compositions when placed in environments which simulate body conditions. Thus, in vitro dissolution of the cortisol antagonist may be sustained for at least 0.75 hours, preferably at least 1 or 2 hours, more preferably at least 3 or 4 hours, particularly at least 6 or 8 hours as contrasted with about 0.5 hours for the marketed immediate release product of ketoconazole. The Examples include a suitable assay for measurement of in vitro dissolution.

A particular advantage of the present invention is the way in vitro dissolution data can be correlated with the time taken in vivo for the cortisol antagonist to reach 50% of the maximum plasma concentration. Thus a reliable relationship has been shown between,

$t_{p0.5}$ - the time taken *in vivo* for the cortisol antagonist to reach 50% of the maximum plasma concentration and

$t_{50\%}$ - the time taken *in vitro* to dissolve (release) 50% of the dose.

For formulations wherein at least one release rate controlling substance is a monoglyceride, in particular a monoglyceride rich in monooleate and/or monolinoleate fatty acyl chains, the relationship can be presented as follows:

$$t_{p0.5} = 1.0 \text{ to } 1.5 + 0.75 \text{ to } 0.95\ t_{50\%}.$$

Example 12 explains that this relationship can be the same for a group of different cortisol antagonists. This correlation means that the number of in vivo experiments can be reduced and suitable candidate formulations identified largely through in vitro work.

As discussed above, cortisol levels in the body tend to rise and peak during the night. Surprisingly, some preferred formulations of the present invention provide a delayed onset to the release of the cortisol antagonist. This has the advantage that the composition can be administered while the patient is still awake e.g. before bed-time, between 9p.m. and 11p.m. and yet the release of cortisol antagonist is delayed and so plasma levels of cortisol antagonist more closely follow the normal circadian pattern of circulating cortisol levels. In this way, a cortisol synthesis inhibitor such as ketoconazole is made available at the most suitable time for counteracting the nightly increase in cortisol synthesis.

Thus, in a preferred embodiment of the present invention, circulating plasma levels of the cortisol antagonist reach pharmaceutically effective levels more than ½ hour, preferably more than 1 hour, more preferably more than 2 hours after administration of the composition. A practical definition of pharmaceutically effective' is provided above in the context of sustained released.

The formulations of the invention will therefore preferably exhibit a 'lag time' between administration and the presence in the plasma of pharmaceutically effective levels. Another way to define and investigate this delayed onset to the release of the cortisol antagonist is to calculate $t_{first}$. the arithmetic mean between the time of the last sample point before appearance in plasma and the time of the first sample point at which the active agent is detected in the plasma. Thus, for example, if samples are analysed every 20 mins from administration and at 300 mins there is no detectable antagonist in the sample but at 320 mins cortisol antagonist is detected, $t_{first}$ will be 310 mins. $t_{first}$ may vary slightly with the detection method used; according to the techniques described herein for example, ketoconazole is detectable in plasma only at concentrations greater than or equal to 100 ng/ml. The compositions of the invention will preferably have a $t_{first}$ in excess of ½ hour, preferably in excess of 1 hour, more preferably in excess of 1½ or even 2 hours. The values for $t_{first}$ are preferably mean values, there being a potential for considerable variation from patient to patient.

The delay may conveniently be achieved due to the time taken by the composition to react to its surroundings, e.g. by taking on water or reacting to a change in pH, and undergo a change of state in order to release the active ingredient(s).

It is easily realised by those skilled in the art that the pharmacokinetic parameters such as $C_{max}$ (peak plasma concentration of cortisol antagonist), $t_{max}$ (time of peak plasma concentration of cortisol antagonist) $t_{first}$ (defined above) and bioavailability of the formulations according to the invention depend on e.g. (1) the choice of cortisol antagonist, (2) the choice of release rate controlling substance in the formulation, (3) the route of administration and (4) the state of the patient. With regard to (1), it can be shown by investigation and comparison of in vitro properties that certain

cortisol antagonists are likely to perform in a similar manner in vivo and thus have similar pharmacokinetic/dynamic properties when formulated, see Examples 8 to 12 herein.

Advantageously, where the cortisol antagonist is ketoconazole, the time taken from administration for the plasma ketoconazole concentration to first exceed 100ng/ml will be in excess of 0.5 hour, preferably in excess of 1 hour and more preferably in excess of 2 or 3 hours. As will be discussed in more detail below, this is conveniently achieved following oral administration of the compositions of the invention. Inter-patient variation can be considerable and the above values represent typical values or mean values for a sample of patients.

As well as the time taken for the cortisol antagonist to reach a threshold circulating concentration, the delayed onset and sustained release of the formulations of the invention can be contrasted with standard immediate release dosage forms such as Nizoral® by reference to the time taken for the circulating concentration of cortisol antagonist to peak. This will typically be more than 2, preferably more than 3, e.g. between 4 and 8 hours after administration.

A typical profile for plasma concentration of a cortisol antagonist administered in a composition according to the present invention has 4 distinct phases. During the first phase, there is minimal release of cortisol antagonist from the formulation and circulating levels of the cortisol antagonist rise negligibly if at all. In the second phase, there is a fairly steep increase in concentration in the plasma. The third phase is a "plateau" phase where circulating levels remain roughly constant. In the final phase, the circulating level drops to the starting concentration. Phases 3 and 4 may not be distinct, instead there may be a gradual decline in plasma concentration after the peak value has been reached. The presence in the plasma profile of a delayed but then rather rapid increase in cortisol antagonist concentration represents a preferred feature of the present invention. An assessment of how rapid is the increase in cortisol antagonist can be made by measuring the time taken from $t_{first}$ to $C_{max}$, this will preferably be less than 7 hours, more preferably less than 6 hours, possibly less than 4 hours but a large variation between patients and different formulations may be observed.

Suitable release rate controlling substances are pharmaceutically acceptable excipients which are capable of modifying the rate at which the cortisol antagonist becomes available for uptake by the body. At least one release rate controlling substance will be a fatty acid ester wherein the fatty acid component is a saturated or unsaturated fatty acid having between 6 and 26 carbon atoms. The release rate controlling substance is central to modulating the pattern of active agent-release in a predictable fashion. The ability of this substance and/or the formulation as a whole which incorporates the substance to undergo a change in state following administration, typically provides a mechanism which allows gradual and sustained release from the composition of the active agent.

The release rate controlling substance will preferably be in the form of a solid or semi-solid matrix, in which, conveniently, the cortisol antagonist may be dispersed. This can be contrasted with simple capsules, films, coatings and the like which may provide, for a time, a barrier between the active ingredient and the body but are not release rate controlling substances as understood by the skilled man working in this area.

In a preferred embodiment of the present invention, the release rate controlling substance will be a lipid, preferably a polar lipid, i.e. compound containing a hydrophobic part and having at least one hydrophilic group. Preferably the release rate controlling substance will comprise a fatty acid.

Examples of saturated fatty acid moieties in the fatty acid esters according to the invention are those selected from the group consisting of moieties of caproic acid, caprylic acid, capric acid, lauric acid, myristic acid, palmitic acid, stearic acid, arachidic acid, and behenic acid.

Examples of unsaturated fatty acid moieties in the fatty acid esters according to the invention are moieties selected from the group consisting of palmitoleic acid, oleic acid, linoleic acid, linolenic acid, and arachidonic acid.

Particularly suitable fatty acid esters for use according to the invention are fatty acid esters which are selected from the group consisting of fatty acid esters of polyhydric alcohols, fatty acid esters of hydroxycarboxylic acids, fatty acid esters of monosaccharides, fatty acid esters of glycerylphosphate derivatives, fatty acid esters of glycerylsulfate derivatives, and mixtures thereof. In those cases where the hydroxy-containing component of the fatty acid ester is polyvalent, the hydroxy-containing component may be partially or totally esterified with a fatty acid component or with mixtures of fatty acid components.

The polyhydric alcohol component of the fatty acid ester for use according to the invention is preferably selected from the group consisting of glycerol, 1,2-propanediol, 1,3-propanediol, diacylgalactosylglycerol, diacyldigalactosylglycerol, erythritol, xylitol, adonitol, arabitol, mannitol, and sorbitol. The fatty acid esters formed from such polyhydric alcohols may be mono- or polyvalent such as, e.g., divalent, trivalent, etc., fatty acid monoesters being preferred. The position of the polyvalent alcohol on which the ester bond(s) is(are) established may be any possible position. In those cases where the fatty acid ester is a diester, triester, etc. the fatty acid components of the fatty acid ester may be the same or different. In a most preferred aspect of the present invention, the polyhydric alcohol component is glycerol.

In those cases where the fatty acid ester for use according to the present invention is formed between a hydroxycarboxylic acid (or a derivative thereof) and a fatty acid (or a derivative thereof), the hydroxycarboxylic acid component of the fatty acid ester is preferably selected from the group consisting of malic acid, tartaric acid, citric acid,

and lactic acid.

As mentioned above, the hydroxy-containing component of a fatty acid ester for use according to the present invention may also be a saccharide, such as a monosaccharide such as, e.g., glucose, mannose, fructose, threose, gulose, arabinose, ribose, erythrose, lyxose, galactose, sorbose, altrose, tallose, idose, rhamnose, or allose. In those cases where the hydroxy-containing component is a monosaccharide, the fatty acid ester is preferably a fatty acid monoester of a monosaccharide selected from the group consisting of sorbose, galactose, ribose, and rhamnose.

The hydroxy-containing component of a fatty acid ester for use according to the invention may also be a glycerylphosphate derivative such as, e.g., a phospholipid selected from the group consisting of phosphatidic acid, phosphatidylserine, phosphatidylethanolamine, phosphatidylcholine, phosphatidylglycerol, phosphatidylinositole, and diphosphatidylglycerol.

Especially interesting compounds having a phospholipid moiety are compounds wherein the fatty acid ester is a fatty acid ester of a glycerylphosphate derivative, and the fatty acid component is selected from the group consisting of lauric acid, myristic acid, palmitic acid, stearic acid, oleic acid, linoleic acid, linolenic acid, and behenic acid. Examples of such useful fatty acid esters are dioleyol phosphatidylcholin, dilauryl phosphatidylcholin, dimyristyl phosphatidylcholin, dipalmitoyl phosphatidylcholin, distearoyl phosphatidylcholin, dibehenoyl phosphatidylcholin, dimyristyl phosphatidylethanolamine, dipalmitoyl phosphatidylethanolsmine, dioleyl phosphatidylglycerol, dilauryl phosphatidylglycerol, dimyristoyl phosphatidylglycerol, dipalmitoyl phosphatidylglycerol, distearoyl phosphatidylglycerol, dipalmitoyl phosphatic acid and mixtures thereof.

Most of the fatty acid esters for use according to the invention are well-known chemical compounds which are commercially available or may be prepared by means of conventional esterification procedures involving e.g. reaction of a fatty acid derivative such as, e.g., the corresponding acid chloride with a hydroxy-containing compound (if necessary protected with suitable protection groups) and subsequently isolating the fatty acid ester, if necessary after removal of any protecting group. Many of the commercially available fatty acid esters are employed in the food and pharmaceutical industries and in general, no steps are taken in order to obtain an approximately 100% pure fatty acid ester. As an example it can be mentioned that glycerylmonooleate (Rylo MG 19®) from Danisco Grindsted Ingredients A/S, Denmark is a very pure product containing about 98% w/w monoesters of which more than about 80% w/w (such as about 92% w/w) is glycerylmonooleate; the remaining monoesters are glycerylmonolinoleate, glyceryl monopalmitate and glyceryl monostearate. The fatty acid ester products for use according to the invention may thus be mixtures of fatty acid esters. An alternative commercially available glycerylmonooleate product which may be used is Rylo MG 20® also from Danisco.

The fatty acid esters may conveniently be a glyceride or a phospholipid, sn-glycerol-3-phosphate which has been esterified by reaction with fatty acids.

Particularly preferred release rate controlling substances are glycerides, mono-, di- and triglycerides with fatty acid residues having between 6 and 22 carbon atoms, more preferably between 16 and 22 carbon atoms. Monoglycerides being rich in unsaturated fatty acid residues having between 16 and 22 carbon atoms are particularly preferred. Diglycerides such as glycerol dioleate may also conveniently be used as a release rate controlling substance in the compositions of the invention. These latter formulations may be based on compositions described in US 5,807,573, which is incorporated herein by reference, and which discloses a glycerol-ester based controlled release composition being a mixture of at least one diacyl glycerol having an unsaturated fatty acid chain containing 16-22 carbon atoms and at least one phospholipid selected from glycerophosphatides and sphingophosphatides. Glycerol dioleate, particularly together with lecithins and/or phosphatidylcholines, are preferred for use in the compositions of the present invention.

Monoglycerides such as monoolein and monoolein rich monoglyceride blends are especially preferred for use in the compositions of the invention. By monoolein is meant a product which consists predominantly of glyceryl monooleate (also known as glycerol monooleate), although various other glycerides of oleic acid and other fatty acids could be present. Hereinafter references to glyceryl monooleate should be construed also as a reference to monoolein and visa versa. Thus preferably the compositions of the invention will include glyceryl monooleate or glyceryl monolinoleate, glyceryl monooleate being preferred.

It has been found that compositions containing a monoglyceride such as glyceryl monooleate preferably also contain a small proportion of a diglyceride such as glycerol diooleate. Commercially available monoglyceride products (e.g. Rylo MG 19®) may contain a small amount of diglyceride e.g. about 0.5-2%, most of the remainder will be monoglyceride with a very small amount of triglyceride possibly also present. Experiments were performed wherein 2-9% diglyceride was added to Rylo MG 19® (this stating material contained 1.0% diglyceride and 98.7% monoglyceride) to provide compositions wherein the monoglyceride content varied from 90.9-97.7%.

The dissolution profile of these compositions was investigated and surprisingly it was found that moderate amounts of diglycerides (more than is generally found in Rylo MG 19®) gave the best results. Thus, in a preferred embodiment, the compositions of the invention will preferably contain in addition to a monoglyceride, 2.5 to 6.5% preferably 3 to 5.3% by weight of a diglyceride. Especially preferred compositions will contain 97 to 94.5% of monoglycerides and 3 to 5.5% diglycerides.

Preferred, polar lipids are those which have fluid, rather than crystalline fatty acid moieties. The fatty acid moieties are thus preferably unsaturated rather than saturated. Compositions of the invention may comprise a mixture of fatty acids and/or fatty acid containing molecules; preferably more than 5 or 10%, more preferably more than 20%, most preferably more than 50%, particularly more than 70% of the fatty acid moieties are unsaturated.

Instead of, but preferably as well as a lipid portion as defined above, the release rate controlling substance may comprise a polymer.

In preferred embodiments of the present invention, polymers act to modify the speed at which the cortisol antagonist is released from the formulation. The polymeric substances may act as an obstruction factor to release of cortisol antagonist, e.g. by hampering the diffusion from the formulation of the dissolved cortisol antagonist. Preferred polymers include polysaccharides. Suitable polymers include alginates, polyacrylic acids (carbopols), chitosan, carboxyvinyl polymers, cellulose and cellulose derivatives such as hydroxypropyl methylcellulose, calcium or sodium carboxymethylcellulose, microcrystalline cellulose, powdered cellulose and starch and derivatives thereof.

Fatty acid esters and mixtures thereof particularly glycerides are preferred release rate controlling substances according to the invention, but the formulations may advantageously additionally comprise a further lipid component, in particular fatty acids having 16 to 26 carbon atoms e.g. oleic acid.

Preferred compositions according to the invention will therefore comprise a fatty acid ester wherein the fatty acid component is saturated or unsaturated fatty acid having between 6 and 26 carbon atoms, particularly a monoglyceride such as monoolein together with oleic acid or sesame oil and/or a cellulose based polymer.

The compositions may also comprise further active ingredients as well as one or more cortisol antagonist. Such additional active ingredients will be selected dependent upon the disease/condition being treated and the patient themselves. Growth hormone, testosterone and oestrogen (or analogous thereof or agents which mimic their activity) may conveniently be co-administered with a cortisol antagonist such as ketoconazole in the treatment of metabolic syndrome. Co-administration does not require all components to be present in a single formulation as the same route of administration may not be appropriate in all cases.

Further ingredients acting as release rate controlling substances may be added as well as other pharmaceutically acceptable carriers, stabilisers and formulation modifiers. Such are well known to the man skilled in the art and include pH modifiers, antioxidants and complexing agents and are exemplified by calcium chloride dihydrate, polyvinyl alcohol, titanium dioxide, tricalcium phosphate, sodium chloride, hydrochloric acid, galactomannan, scleroglucan, citric acid, $NaHPO_4$ and trisodium citrate dihydrate.

In its simplest form however, the invention provides a composition comprising at least one release rate controlling substance and a pharmaceutically effective amount of a cortisol antagonist wherein at least one release rate controlling substance is a fatty acid ester wherein the fatty acid component is a saturated or unsaturated fatty acid having between 6 and 26 carbon atoms, and the composition is adapted for oral administration.

Compositions according to the invention may comprise between 1mg and 1000mg of cortisol antagonist, typically between 50mg and 800mg, preferably between 200mg and 600mg (e.g. 200 mg of ketoconazole). This may constitute between 1 and 99% by weight of the total composition, typically between 10 and 80%, preferably between 25 and 75% by weight of the total composition.

Cortisol overactivity has been implicated in a large number of medical conditions and the compositions of the invention may be used to treat any of these. Therapeutic use of cortisol lowering pharmaceuticals has been considered for the treatment of stress (Med. Hypothesis 13, 31-44, 1984) and anti-cortisol therapy has been suggested for the treatment of an extensive list of diseases such as Cushing's disease/syndrome and depressive diseases, (Psychoneuroendocrinology 22 (suppl. 1) S3-10, 1997). Further diseases which may be treated with the compositions of the invention include androgen related diseases such as prostate cancer, hirsutism and polycystic ovary syndrome. Thus in a further aspect of the present invention is provided a composition according to the invention as defined herein for use in the treatment of any condition related to elevated cortisol activity and/or androgen activity. Suitable conditions will generally be those where elevated circulating levels of cortisol and/or androgens are at least partly responsible for observed symptoms or constitute a risk factor for other conditions.

However, the compositions of the invention are particularly suitable for the prevention or treatment of metabolic syndrome or diabetes mellitis type II or symptoms and complications thereof. Thus in a further preferred embodiment the invention provides a composition for controlled release of a cortisol antagonist, wherein said composition is adapted for oral administration, comprising at least one release rate controlling substance, wherein at least one of the release rate controlling substances is a fatty acid ester wherein the fatty acid component is a saturated or unsaturated fatty acid having between 6 and 26 carbon atoms, and a pharmaceutically effective amount of a cortisol antagonist for use in the treatment of metabolic syndrome or diabetes mellitis type II.

"Treatment" refers to total or partial alleviation of at least one of the symptoms associated with the disease or complications thereof and reference to metabolic syndrome and diabetes mellitus type II may be construed as a reference to the symptoms and complications associated therewith. In a further aspect, the invention provides a method

of treating metabolic syndrome or diabetes mellitis type II and symptoms and complications associated therewith which method comprises administering a composition according to the present invention as defined herein. The compositions of the invention are of use in the treatment of mammals, particularly humans.

Glyceryl monooleate has been described in the past for use in controlled release formulations. These previously described uses typically relate to formulations which are to be positioned in body cavities or sites, in particular in a periodontal pocket, WO92/09272 and US 5,262,164. However it has now surprisingly been found that compositions of the invention which include release rate controlling substances such as glyceryl monooleate, can successfully be administered orally. The cortisol antagonist is released in the desired time related manner as the composition passes through the gastro-intestinal (GI) tract and is thus available for absorption.

It was surprisingly found that such compositions when administered orally gave desirable plasma levels of cortisol antagonist. Absorption of active ingredient may take place at any point along the GI tract. Known methods can be used, e.g. by providing enteric coated capsules with acidic buffer to target the mid to lower GI tract. However it has surprisingly been found that excellent absorption of the cortisol antagonist can be achieved in the stomach over an extended time period. Polymers which swell may advantageously be added to the composition in order to encourage retention of the composition in the stomach. The stomach discharges its contents through the pylorus into the small intestine. If the tablets are larger than the pylorus, they can stay in the stomach for a long time. For the sake of ease of swallowing, the tablets should swell in the stomach and attain a large size quickly. Alternatively, "floating" formulations may be prepared which tend to be retained in the stomach for a long time, floating on the gastric contents. Floating formulations may be prepared by adding gel-forming hydrocolloids such as HPMC and fillers of low density in a tablet.

It has very surprisingly been observed that the cortisol antagonist when formulated according to the present invention may also be absorbed in the intestine. Given the higher pH of the intestine compared to the stomach, absorption of, e.g. ketoconazole may be expected to be negligible in the intestine. However, this finding presents a further benefit of the present invention as absorption may be even further extended if it can take place along all or most of the GI tract.

Lower plasma levels of active agent are generally observed when absorption has taken place in the intestine, in certain circumstances this may be desirable, for example when lower peak doses over extended periods are required. Lymphatic uptake may be relevant to intestinal absorption and the compositions may be designed to enhance this, possibly by inclusion of oleic acid. Aside from the properties of the basic formulations which may provide surprising intestinal uptake (e.g. formulation I in Example 7), an enteric coating may be provided to enhance intestinal rather than gastric uptake.

The formulations of the invention are adapted for oral administration. The present invention thus provides a composition for controlled release of a cortisol antagonist comprising at least one release rate controlling substance and a pharmaceutically effective amount of said cortisol antagonist, wherein at least one of the rate controlling substance is a fatty acid ester wherein the fatty acid component is a saturated or unsaturated fatty acid having between 6 and 26 carbon atoms, said composition being adapted for oral administration and preferably (particularly where the cortisol antagonist is ketoconazole) allowing or facilitating absorption of the cortisol antagonist to take place substantially in the stomach. For other cortisol antagonists, the composition may conveniently allow or facilitate absorption of the cortisol antagonist in the intestine.

Absorption may take place all along the GI tract but for cortisol antagonists like ketoconazole, the majority (i. e. more than 50%, preferably more than 60%, more preferably more than 80 or 90% of cortisol antagonist which circulates in the plasma will preferably have been taken up in the stomach. Uptake may be by the lymphatic or hepatic routes.

Suitable methods for preparing compositions for oral administration are known to the skilled man. Formulations may be presented in tablet, capsule, powder, syrup, solution or suspension form, tablets and capsules being preferred. The compositions of the invention, which are preferably in solid or semi-solid form, may conveniently be covered in a gelatin or similar capsule for oral administration. Capsules may be hard such as the gelatin capsule of size 000, 43.000 Capsugel (Bornem, Belgium) or soft, hard gelatin capsules being preferred. Suitable encapsulation and tableting techniques are described by Takada K. et al in Encycl. Controlled Drug Deliv. (1999) 2 698-728. Generally, the capsules function merely as a container for the composition of active ingredient and release rate controlling substance. The capsule will become permeable/disintegrate or detach soon after administration of the dosage form, typically in 5-15 mins and is therefore not acting as a release rate controlling substance as defined herein.

As discussed above, the compositions according to the invention is preferably administered in encapsulated or tablet form. This formulation preferably retains a substantially unitary form on administration and throughout the controlled release of the cortisol antagonist. The formulation as administered will typically vary in size from 0.5 to 1.5 $cm^3$, preferably about 1 $cm^3$, to which any capsule contributes negligible volume. The formulation may break into a few pieces, e.g. 2 or 3 but each piece will remain substantially spherical. The fact that the formulation may remain more or less intact (after loss of the capsule where relevant) is beneficial when the formulation is designed to be retained in the stomach. It also means that the composition has minimal contact with the mucosa of the GI tract, a substantially

spherical and rather large dosage form will only have a very small percentage of its surface area in contact with the mucosal lining. Thus, any bioadhesive properties of the release rate controlling substance or any other components of the composition will be largely irrelevant and the components of the formulation are not chosen for their bioadhesive properties. Bioadhesive formulations generally contain small particles or are otherwise designed to maximise contact with the mucosa.

The compositions may be formulated by any of the methods known in the art and suitable methods are described in the Examples. Conveniently, a homogenous dispersion of the cortisol antagonist and release rate controlling substance is prepared above the melting point of the composition which may then subsequently be solidified. Alternatively, the release rate controlling substance may be frozen and then crushed to a fine powder before being mixed with the cortisol antagonist and any other excipients in dry form to make a homogenous formulation.

Thus, in a further aspect, the present invention provides a method of making a composition for controlled release of a cortisol antagonist, wherein said composition is adapted for oral administration, which method comprises mixing said cortisol antagonist with a release rate controlling substance, preferably to provide a homogeneous formulation, and wherein at least one of the release rate controlling substances is a fatty acid ester wherein the fatty acid component is a saturated or unsaturated fatty acid having between 6 and 26 carbon atoms.

Glycerol monooleate and similar compounds have been disclosed for use as bioadhesives. Examples of publications illustrating this are given in US 5,955,502 to Nielsen *et al*. and WO 98/47487 to Nielsen *et al*.

WO 95/26715 to Hansen et al. describe the use of fatty acid esters as bioadhesive substances for application to or through mucosa including gastrointestinal mucosa. WO 98/47487 to Nielsen et al. describe bioadhesive compositions comprising certain structurants as additives to a substance, preferably a fatty acid ester, which is capable of forming one or more lyotropic liquid crystals. WO 97/13528 to Nielsen et al. describe a bioadhesive composition comprising antiherpes virus agents compounded in an bioadhesive matrix based on a fatty acid ester capable of forming a lyotropic liquid crystalline phase.

The invention will be further described with reference to the following non-limiting Examples in which:

**Figure 1** shows dissolution rate profiles of compositions according to the invention as compared to Nizoral® (Fungoral);
**Figure 2** shows plasma profiles in healthy human subjects according to Example 7 for formulation E in Table 3;
**Figure 3** shows the mean plasma profile in the subjects for formulation E in Table 3 as compared to Nizoral;
**Figure 4** shows plasma profiles in healthy human subjects according to Example 7 for formulation F in Table 3;
**Figure 5** shows the mean plasma profile in the subjects for formulation F in Table 3 as compared to Nizoral;
**Figure 6** shows plasma profiles in healthy human subjects according to Example 7 for formulation I in Table 3;
**Figure 7** shows the mean plasma profile in the subjects for formulation I in Table 3 as compared to Nizoral;
**Figure 8** shows plasma profiles in healthy human subjects according to Example 7 for formulation J in Table 3;
**Figure 9** shows the mean plasma profile in the subjects for formulation J in Table 3 as compared to Nizoral;
**Figure 10** shows plasma profiles in healthy human subjects according to Example 7 for formulation A in Table 3;
**Figure 11** shows the mean plasma profile in the subjects for formulation A in Table 3 as compared to Nizoral;
**Figure 12** shows plasma profiles in healthy human subjects according to Example 7 for formulation B in Table 3;
**Figure 13** shows the mean plasma profile in the subjects for formulation B in Table 3 as compared to Nizoral;
**Figure 14** shows an in vitro/in vivo correlation for formulations J, E and F according to Table 3 between the in vitro time to dissolve (release) 50% ($t_{50\%}$) of the dose and the in vivo parameter time to reach 50% of the maximum plasma concentration ($t_{p0.5}$). Nizoral® 200 mg is used as reference (data for the immediate release (IR) product from Huang, Y.C. et al., Antimicrob Agents Chemother. 1986. 30(2): p. 206-10 and Daneshmend, T.K. *et al*. J. Antimicrob. Chemother., 1986. 18(2): p. 289-91). The dashed curves indicate 95% confidence interval of the linear regression (solid line);
**Figure 15** shows dissolution rate profiles of compositions according to Example 9;
**Figure 16** shows dissolution rate profiles of compositions according to Example 10; and
**Figure 17** shows dissolution rate profiles of compositions according to Example 11.

## EXAMPLES

Throughout the Examples, the following materials are used:

Table 1

| Excipients | | | |
|---|---|---|---|
| Excipients | Abbreviate | Supplier | Quality |
| Glycerol Monooleate | GMO | Danisco Ingr. | |
| Glycerol Dioleate | GDO | Danisco Ingr | |
| Phosphatidyl Choline | PC | Lucas Mayer | |
| Sesamolja | SO | Apoteksbolaget | Ph Eur |
| Ketoconazole | KC | Gufic Health Care Ltd | |
| Oleic Acid | OA | Kebo Lab | |
| Hydroxy Propyl-Methyl Cellulose | HPMC | Sigma | USP |
| Sodium Carboxy-Methyl Cellulose | SCMC | Sigma | USP |
| Carbopol 934 P | CP | BF Goodrich | Ph |
| Grade Sucros Sodium alginate/ Kelgin LV | Na-Alg | Sigma Kelco | USP |
| Calcium chloride dihydrate | $CaCl_2$ | Apoteksbolaget | Ph Eur |
| Polyvinyl alcohol | PVA | Sigma | USP |
| Titanium dioxide | TiO | Sigma | |
| TriCalcium Phosphate | TriCaP | Merck | BP |
| Water for injection | WFI | Braun | Ph Eur |
| Sodium chloride | NaCl | Merck | pa |
| Hydrocloric acid, 37% | HCl | Merck | pa |
| Galactomannan Scleroglucan Citric acid | CA | Pentapharm Ltd Alban Muller Int. Apoteksbolaget | Ph Eur |
| Di-Sodiumhydrogen phophatedihydrate | $NaHPO_4$ | Merck | pa |
| Tri-Sodium citrate Dihydrate | NaCA | Merck | pa |

**Solutions**

Simulated Gastric Fluid,
2.0 g NaCl + 7 ml HCl, fuming 37%, dilute to 1000 ml with distilled water.
Sodium-citrate buffer pH 1
21.1ml 0.1M NaCA and 88.9ml 0.1M HCl were mixed and adjusted to pH 1.0 with 1M NaOH.
Citrate-phosphate buffer pH 7
7.0 ml 0.1M CA and 33.0 ml 0.2M $NaHPO_4$ were mixed.

**Others**

Hard gelatine capsules, Size 000, Capsugel

**EXAMPLE 1**

Ketoconazole, USP was compounded in melted glycerol dioleate at 40°C in the following proportions:

| Component: | Weight%: |
|---|---|
| Ketoconazole | 20 |
| Glycerol diooleate | 48 |
| Phosphatidylcholine | 32 |

The above formulation can be prepared in several alternative ways. One way is as follows: phosphatidylcholine is dissolved in ethanol (95%) to which glycerol diooleate is added. The so obtained mixture was freeze dried until a constant weight was obtained. The mixture was heated to 40°C and ketoconazole was added. Subsequently, the formulation was processed until a homogenous mixture was obtained and the mixture was filled in hard gelatin capsules (43,000 Capsugel®). The homogeneity of ketoconazole was assessed by means of HPLC.

## EXAMPLE 2

The solubility of ketoconazole in glycerol monooleate was assessed at 37°C according to the following method. 2g samples of ketoconazole (USP) and glycerol monooleate were mixed together in various proportions and put on a magnetic stirrer at 37°C. Samples containing 1, 3 and 5 wt. % of ketoconazole were investigated for the observation of ketoconazole crystals. Samples containing 1 wt. % ketoconazole were completely dissolved after less than 18 h but more than 2 h. Samples containing 3 wt % ketoconazole were almost homogeneous after 48 h, after which no further change of the sample took place. In samples containing 5 wt. % ketoconazole, the appearance of ketoconazole crystals showed that it was not completely dissolved. Thus, at 37°C ketoconazole has a limited solubility in glycerol monooleate. It is estimated to be between 1 and 3 wt. %.

Ketoconazole, USP was compounded in melted GMO at 40°C in the following proportions:

| Component: | Weight%: |
|---|---|
| Ketoconazole | 20 |
| Glycerol monooleate | 80 |

The above formulation can be prepared in different ways. One way is as follows: ketoconazole was added to melted glycerol monooleate and the mixture was stirred at 40°C until a homogenous mixture was obtained. One gram of the mixture was subsequently filled into hard gelatin capsules (43.000).

Alternatively, frozen glycerol monooleate was mortared to a powder to which ketoconazole was added. The so obtained mixture was subsequently processed in the cold until a homogenous mixture was obtained. 1g of formulation was put in each capsule. The capsules were stored in the refrigerator until the dissolution-test was done.

Homogeneity of ketoconazole was assessed by means of HPLC.

## EXAMPLE 3

Ketoconazole, USP was compounded in glycerol monooleate and sesame oil in the following proportions:

| Component: | Weight%: |
|---|---|
| Ketoconazole | 20 |
| Glycerol monooleate | 70.4 |
| Sesame oil | 9.6 |

## EXAMPLE 4

The solubility of ketoconazole in oleic acid was assessed at ambient room temperature using the method described in Example 1. Samples containing 5, 10, 15 and 33 wt. % of ketoconazole were mixed with oleic acid and the appearance of undissolved ketoconazole was investigated after 24 and 48 h. After 24 hours samples containing 5 wt. % ketoconazole were completely dissolved and after 48 h samples containing 10 wt. % of ketoconazole were completely homogenous and all ketoconazole was dissolved. Thus the solubility of ketoconazole in oleic acid is estimated to be about 10 wt. %.

Ketoconazole, USP, was compounded in various mixtures of glycerol monooleate and oleic acid in the following proportions:

| Component: | Weight%: |
|---|---|
| Ketoconazole | 20 |
| Glycerol monooleate | 70 |
| Oleic acid | 10 |

| Component: | Weight%: |
|---|---|
| Ketoconazole | 20 |

(continued)

| Component: | Weight%: |
|---|---|
| Glycerol monooleate | 60 |
| Oleic acid | 20 |

**EXAMPLE 5**

The following compositions of the invention were also prepared:

| Component: | Weight%: |
|---|---|
| Ketoconazole | 20 |
| Glycerol monooleate | 70.4 |
| Sesame oil | 9.6 |

| Component: | Weight%: |
|---|---|
| Ketoconazole | 20 |
| Glycerol monoloeate | 78 |
| Hydroxypropyl methylcellulose | 2 |

| Component: | Weight%: |
|---|---|
| Ketoconazole | 20 |
| Glycerol monoloeate | 79 |
| Sodium alginate | 1 |

With sodium alginate (Kelgin-LV, Kelco):

| Component: | Weight%: |
|---|---|
| Ketoconazole | 20 |
| Glycerol mondloeate | 58 |
| Sodium alginate | 1 |
| Oleic acid | 21 |

With sodium alginate (Kelgin-LV, Kelco) and calcium chloride:

| Component: | Weight%: |
|---|---|
| Ketoconazole | 20 |
| Glycerol monoloeate | 58 |
| Sodium alginate | 1 |
| Calcium chloride | 2 |

Melted and ground glycerol monooleate

| Component: | Weight%: |
|---|---|
| Ketoconazole | 20 |
| Glycerol monoloeate | 56 |
| Sodium alginate | 1 |
| Calcium chloride | 2.5 |
| Oleic acid | 21 |

With hydroxypropyl methylcellulose (Sigma, USP):

| Component: | Weight%: |
|---|---|
| Ketoconazole | 20 |
| Glycerol monoloeate | 74 |
| Sodium alginate | 1 |
| Calcium chloride | 3.3 |
| Hydroxypropyl methylcellulose | 2 |

With sodium carboxymethylcellulose (Sigma, USP):

| Component: | Weight%: |
|---|---|
| Ketoconazole | 20 |
| Glycerol monoloeate | 75 |
| Sodium alginate | 1 |
| Calcium chloride | 2.5 |
| Sodium carboxymethylcellulose | 2 |

With carbopol 934P (BF Goodrich):

| Component: | Weight%: |
|---|---|
| Ketoconazole | 20 |
| Glycerol monoloeate | 74 |
| Sodium alginate | 1 |
| Calcium chloride | 2.6 |
| Carbopol | 2 |

## EXAMPLE 6

The dissolution profile of ketoconazole from the matrices of release rate controlling substances filled in hard gelatin capsule was determined for some of the above compositions (and others) using a USP apparatus 1 equipped with a UV-spectroscopy monitoring at 254 nm.

Formulations were prepared by one of the following two methods.

Using frozen GMO

GMO and a mortar were placed in the freezer overnight. GMO was then crushed to a quite fine powder. The GMO-powder was mixed with dry substances as KC, NaAlg, $CaCl_2$, HPMC, SCMC and/or CP to a homogenous formulation. 1g of formulation was put in each capsule. The capsules were stored in the refrigerator until dissolution-test was done.

Using melted GMO

GMO was melted at 40°C and mixed with substances as KC, OA, NaAlg, $CaCl_2$, HPMC, SCMC and/or CP to a homogenous formulation. The formulation was cooled in the refrigerator until the formulation becomes thicker and easy to inject with a syringe. The capsules were filled with 1g of the formulation and stored in the refrigerator until dissolution-test was done.

Solutions used as media were prepared as follows. USP XXIII Simulated Gastric Fluid (SGF) without pepsin: 2.0 g NaCl+7 ml HCl, fuming 37%, dilute to 1000 ml with distilled water. The resulting SGF has pH 1.2. Sodium-citrate buffer pH 1:21.1 ml 0.1M NaCA and 88.9 ml 0.1M HCl were mixed and adjusted to pH 1.0 with 1M NaOH. Citrate-phosphate buffer pH 7:7.0 ml 0.1M CA and 33.0 ml 0.2M $NaHPO_4$ were mixed.

In addition, visual studies of the swelling ability and buoyancy of the above compositions and others were performed. The visual studies were made in flasks containing 50 ml SGF, pre-heated to 37°C. The flasks were placed on a shaking board at a slow rotating speed of 150-200 rpm and the capsules were dropped down. A timer was started and the buoyancy and swellings were studied.

Results of these experiments are summarised in Table 2 below and in Fig. 1.

Table 2

| Composition of formulation (%, w/w) | Time (h) 50% released | Visual study of capsule in SGF at 37°C | |
|---|---|---|---|
| | | **Swellable <30 min** | **Buoyancy** |
| GMO/KC 80/20 | 0.8 | Little | Floats |
| GMO/KC/OA<br>60/20/20[1]<br>70/20/10' | <br>2.2<br>1.8 | <br>Little<br>Little | <br>Floats<br>Floats |
| GMO/KC/HPMC<br>78/20/2[1] | <br>1.6 | <br>Little | <br>Floats |
| GMO/KC/HPMC/TriCaP<br>67/20/3/10 | | <br>Little | <br>Floats |
| GMO/KC/OA/HPMC<br>67/20/11/2 | | <br>Little | <br>Floats |
| GMO/KC/OA/HPMC/$TiO_2$<br>57/20/11/2/10 | | <br>Little | <br>Sinks |
| GMO/KC/OA/HPMC/TriCaP<br>63/20/5/2/10<br>57/20/11/2/10 | | <br>Little<br>Little | <br>Floats<br>Sinks |
| GMO/KC/HPMC/TriCaP/Scleroglucan<br>61/20/2/15/2.6 | | <br>Little | <br>Sinks |
| GMO/KC/HPMC/TriCaP/Galactomann.<br>60/20/2/15/3 | | <br>Little | <br>Floats |
| GMO/KC/SCMC<br>78/20/2 | | <br>Little | Sinks first<br>then floats |
| GMO/KC/SO<br>70.4/20/9.6 | <br>1.6 | Not<br>applicable<br>(breaks) | <br>Floats |
| GMO/KC/Carbopol<br>78/20/2 | | <br>Little | <br>Floats |
| GMO/KC/NaAlg 79/20/1[2] | 1.5 | Little | Floats |

[1] = made using melted GMO
[2] = made using frozen GMO

Table 2 (continued)

| Composition of formulation (%, w/w) | Time (h) 50% released | Visual study of capsule in SGF at 37°C | |
|---|---|---|---|
| | | Swellable <30 min | Buoyancy |
| GMO/KC/NaAlg/OA 58/20/1/21[1] | 2.0 | Little | Floats |
| GMO/KC/NaAlg/OA/HPMC/Tri-CaP 56/20/1/11/2/10 / | | Little | Sinks |
| GMO/KC/NaAlg/$CaCl_2$ 76/20/1/2[1] 76/20/1/2[2] | 2.1 | Little | Floats |
| GMO/KC/NaAlg/$CaCl_2$/OA 56/20/1/2.5/21[1] | 3.5 | Little | Floats |
| GMO/KC/NaAlg/$CaCl_2$/HPMC 74/20/1/3.3/2[2] | 5.4 | Little | Floats |
| GMO/KC/NaAlg/$CaCl_2$/HPMC/PVA 70/20/1/2.5/2/5 | | Little | Floats |
| GMO/KC/NaAlg/$CaCl_2$/HPMC/$TiO_2$ 70/20/1/2.5/2/5 | | Not applicable (breaks) | Floats |
| GMO/KC/NaAlg/$CaCl_2$/HPMC/Sucros 70/20/1/2.6/2/5[1] | | Little | Floats |
| GMO/KC/NaAlg/$CaCl_2$/SCMC 75/20/1/2.5/2[2] | 5.1 | Little | Floats |
| GMO/KC/NaAlg/$CaCl_2$/Carbopol 74/20/1/2.6/2 | 4.8 | Little | Floats |
| GDO/PC/KC 48/16/20 | 6.5 | Little | Floats |

[1] = made using melted GMO
[2] = made using frozen GMO

**EXAMPLE 7**

In order to determine the single oral dose, safety, tolerability and pharmacokinetic profile of six formulations (200 mg ketoconazole), a pilot, Phase I, three-way crossover, single evening oral dose, alternating group study was performed on twelve human volunteers. The dose was administered as hard gelatin capsules. Blood samples were drawn at pre-dose, 0.5, 1, 1.5, 2, 2.5, 3, 4, 6, 6, 12, 16 and 18 hours post-dose.

Formulations according to Table 3, were filled into hard gelatin capsules (43,000). Formulations A and B were administered as enteric coated hard gelatin capsules using Eudragit as a coating material. Thus, these can be considered 'intestinal' formulations, i.e. they are targeted to release the active agent for absorption in the intestines. The enteric coat is designed to resist the gastric juice and therefore the $t_{max}$ is increased beyond that which would be caused by the GMO and other release rate controlling substances. The other formulations are 'gastric' formulations in that they are not made resistant to gastric juice.

Coating was performed on filled capsules. *In vitro* release as described in Example 6 in which firstly coated capsules were put in simulated gastric media (pH 1.2) for 2 hours and then changing the media to simulated intestinal fluid (pH 7.7) showed dissolution of ketoconazole in the latter but not in the acid media. As expected with ketoconazole (being soluble at pH less than 3) the amount of dissolved ketoconazole was substantially as compared to dissolution of ketoconazole, compounded in the invention, from non-coated capsules.

The method of determining levels of ketoconazole in the plasma was based on that described by Kay Hay Yuen and Kok Khiang Peh, Journal of Chromatography B, 715 (1998) 436-440.

Results are shown in Table 3 below and in the figures.

Table. 3

| Formulation Reference Letter: Composition | Mean time of plasma ketoconazole conc.> 100 ng/ml (min ± sd) |
|---|---|
| E: GMO/HPMC/TriCaP/KC (63/2/15/20) | 250±95 (n=6) |
| F: GMO/NaAlg/KC (79/1/20) , | 180±105 (n=6) |
| I: GMO/OA/HPMC/NaAlg/TriCaP/KC (52/2/1/20/20) | 220±135 (n=6) |

Table. 3 (continued)

| **Formulation Reference Letter: Composition** | **Mean time of plasma ketoconazole conc.> 100 ng/ml (min ± sd)** |
|---|---|
| J: GMO/KC (80/20) | 195±130 (n=6) |
| A: GMO/OA/KC (70/20/10) | 360±85 (n=5) |
| B: GMO/OA/KC (59/21/20) | 345±225 (n=6) |

Moreover, none of the subjects reported any adverse effects. Gastrointestinal irritation is a common adverse effect with Nizoral.

Some of the results show substantial uptake of ketoconazole in the intestine, see subject 1 formulation F and subject 8 formulations E and I. These formulations were not targeted to the intestine and yet plasma levels of ketoconazole are of the same magnitude as would be expected for individuals treated with formulations targeted for the intestine. This intestinal uptake is surprising but may be beneficial in certain circumstances. A formulation which allowed intestinal uptake could be administered together with one which provided gastric uptake.

The pharmacokinetic properties of these formulations have been further investigated and the results are given in Tables 4 and 5 below.

Table 4

| **PARAMETER** | **FORMULATION** | | | | | |
|---|---|---|---|---|---|---|
| | **A** | **B** | **E** | **F** | **I** | **J** |
| **N** | 5 | 6 | 6 | 6 | 6 | 6 |
| **$C_{max}$ (ng/mL) ±SD** | 792,20 ±361.568 | 959,72 ±799,452 | 2278,35 ±1358,281 | 2074,00 ±885,778 | 1900,58 ±1092,738 | 2550,05 ±934,900 |
| Min | 472,70 | 263,80 | 876,90 | 880,10 | 721,10 | 1048,90 |
| Median | 743,10 | 674,30 | 1898,20 | 2002,20 | 1662,65 | 2415,40 |
| Max | 1403,50 | 2300,50 | 4715,90 | 3369,90 | 3952,30 | 3616,80 |
| CV% | 45.6 | 83,3 | 59,6 | 42,7 | 57,5 | 36,7 |
| **$t_{max}$ (h) ±SD** | 8,80 ±1,789 | 8,00 ±2.191 | 6,17 ±1,835 | 5,50 ±2,168 | 7,67 ±0,816 | 5,75 ±1,782 |
| Min | 8,00 | 6,00 | 3,00 | 3,00 | 6,00 | 2,50 |
| Median | 8,00 | 8,00 | 6,00 | 5,00 | 8,00 | 6,00 |
| Max | 12,00 | 12,00 | 8,00 | 8,00 | 8,00 | 8,00 |
| CV% | 20,3 | 27,4 | 29,8 | 39,4 | 10,6 | 31,0 |
| **AUC(0-tz) (ng. h/mL) ±SD** | 4312,15 ±3130,644 | 3807,66 ±2982,569 | 12068,10 ±9082,754 | 10236,20 ±3918,934 | 10447,79 ±8742,368 | 12500,44 ±4408,601 |
| Min | 892,50 | 527,60 | 3985,70 | 3689,79 | 2973,87 | 4561,06 |
| Median | 4660,58 | 3851,25 | 10125,53 | 10265,18 | 7639,92 | 13165,88 |
| Max | 9091,27 | 8524,96 | 29178,95 | 14546,73 | 27340,06 | 16707,76 |
| CV% | 72,6 | 78,3 | 75,3 | 38,3 | 83,7 | 35,3 |
| AUC (are under the curve) = area under the plasma/blood concentration-time curve from time zero to time t ($AUC_{ot}$) where t is the last time point with measurable concentration. | | | | | | |

Table 5

| PARAMETER | E | F | I | J |
|---|---|---|---|---|
| $t_{50\%}$ (h) | 4,23 | 2,44 | 18,41[a] | 1,87 |
| $t_{first}$ (h) ± SD | 3,58 ±1,23 | 2,58 ±1,48 | 3,25 ±1,93 | 2,75 ±1,782 |
| $t_{last}$ (h) ±SD | 15,2 ±1,84 | 14,5 ±1,22 | 14,7 ±1,63 | 14,7 ±1,07 |
| $t_{>100}$ (h) ±SD | 9,50 ±3,55 | 9,67 ±2,56 | 9,33 ±4,71 | 10,1 ±2,56 |
| $t_{max}$ (h) ±SD | 6,17 ±1,835 | 5,50 ±2,618 | 7,67 ±0,816 | 5,75 ±1,782 |
| $C_{max}$ (ng/mL) ±SD | 2278,35 ±1358,28 1 | 2074,00 ±855,788 | 1900,58 ±1092,738 | 2550,05 ±934,900 |
| AUC (0-tz) (ng.h/mL) ± SD | 12068,10 ±9082,75 4 | 10236,20 ±3918,934 | 10447,79 ±8742,386 | 12500,44 ±4408,601 |

[a]only about 50% is expected to be released at equilibrium.

The arithmetic means used in the above tables are defined as follows:

$t_{50\%}$ is the time required for half of the dose to be released in vitro.

$t_{>100}$ is the time which the plasma level reached above the limit of detection.

$t_{first}$ is defined as the arithmetic mean between the time of the last sample point before appearance of ketoconazole in plasma and the time of the first sample point at which ketoconazole is detected in the plasma.

$t_{p0.5}$ is the time it takes to reach half the maximum plasma concentration.

$t_{last}$ is analogously to $t_{first}$ defined as the arithmetic mean between the time of the last sample point with detectable amount of ketoconazole and the first time at which sample point ketoconazole plasma concentration is below the limit of detection (<100 ng/ml).

$T_{max}$ is defined according to the standard use in pharmacokinetic studies i.e., as the time at which maximum plasma concentration $C_{max}$ was observed.

$C_{max}$ (ng/mL) is defined as the highest plasma level determined.

**EXAMPLE 8**

Modified release compositions J, E, and F containing 200 mg ketoconazole according to Example 7 were tested for *in vitro*/*in vivo* correlation between the in vitro time to dissolve (release) 50% of the dose and the in vivo parameter time to reach 50% of the maximum plasma concentration. This is known to those skilled in the art as a level C functional relationship that allows prediction of *in vivo* behaviour from dissolution data. The results are shown in Figure 14. From the linear regression of the data it is found that

$$t_{p0.5} = 1.203 + 0.85\, t_{50\%}$$

$t_{p\,0.5}$ = time to reach 50% of maximum in vivo plasma concentration
$t_{50\%}$ = time to release 50% of dose *in vitro*.

**EXAMPLE 9**

Compositions as J, E, and F in Example 7 but instead of ketoconazole containing the cortisol antagonist aminoglutethimide (CAS registry number 125-84-8) were compounded according to Example 2. The formulations were filled into hard gelatin capsules and subsequently tested for *in vitro* dissolution in simulated gastric fluid according to Example 6 with Orimeten® 250 mg as reference immediate release product. The results are shown in Figure 15.

Table 6

| FORMULATION | COMPOSITION (w/w %) | | | | |
|---|---|---|---|---|---|
| | Aminoglutethimide | GMO | Na-alg. | HPMC | TriCaP |
| J | 20 | 80 | - | - | - |
| F | 20 | 79 | 1 | - | - |
| E | 20 | 63 | - | 2 | 15 |
| Reference immediate release drug product | | | | | |
| Orimeten® tablets 250 mg (aminoglutethimide) | | | | | |

**EXAMPLE 10**

Compositions as J, E, and F in Example 7 but instead of ketoconazole containing the cortisol antagonist cimetidine (CAS registry number 51481-61-9) were compounded according to Example 2. The formulations were filled into hard gelatin capsules and subsequently tested for in vitro dissolution in simulated gastric fluid according to Example 6 with Acinil® 200 mg as reference immediate release product. The results are shown in Figure 16.

Table 7

| FORMULATION | COMPOSITION (w/w %) | | | | |
|---|---|---|---|---|---|
| | Cimetidine | GMO | Na-alg. | HPMC | TriCaP |
| J | 20 | 80 | - | - | - |
| F | 20 | 79 | 1 | - | - |
| E | 20 | 63 | - | 2 | 15 |
| Reference immediate release drug product | | | | | |
| Acinil® tablets 200 mg (cimetidine) | | | | | |

**EXAMPLE 11**

Compositions as J, E, and F in Example 7 but instead of ketoconazole containing the coilisol antagonist metyrapone (CAS registry number 54-36-4 were compounded according to Example 2. The formulations were filled into hard gelatin capsules and subsequently tested for in vitro dissolution in simulated gastric fluid according to Example 6 with Metopiron® 250 mg as reference immediate release product. The results are shown in Figure 17.

Table 8

| FORMULATION | COMPOSITION (w/w %) | | | | |
|---|---|---|---|---|---|
| | Metyrapone | GMO | Na-alg. | HPMC | TriCaP |
| J | 20 | 80 | - | - | - |
| F | 20 | 79 | 1 | - | - |
| E | 20 | 63 | - | 2 | 15 |
| Reference immediate release drug product | | | | | |
| Metopiron® tablets 250 mg (metyrapone) | | | | | |

**EXAMPLE 12**

The in vitro release of immediate release products Orimeten®, Acinil® and Metopiron® are similar to that of Nizoral®, i.e time to release 50% of the drug is less or much less than 30 min (for the samples in Figures 15, 16, and 17 it is 10 min).

By way of example, Acinil® immediate release product is characterised by 70% bioavailibility and the time to reach peak plasma concentration in humans is 1-2 hours. Thus the pharmacokinetic parameters are similar to Nizoral®. By a further example, Orimeten® immediate release product is characterised by 90% bioavailibility and the time to reach peak plasma concentration in humans is less than or equal to 2 hours. Thus the pharmacokinetic parameters are similar to Nizoral®. Metopiron® immediate release product is characterised by a fast absorption from the gastrointestinal tract with the time to reach peak plasma concentration in humans being 1 hour after a single 750 mg dose. Thus this pharmacokinetic parameter is similar to Nizoral®.

It is therefore reasonable to expect the three modified release formulations J, E, and F for the cortisol antagonists aminoglutethimide, cimetidine, and nietyrapone, respectively to show a similar *in vitro*/*in vivo* correlation as do ketconazole formulations J, E, and F shown in Example 8 (Figure 14). The results of using this relationship is presented Table 9.

Table 9

| CORTISOL ANTAGONIST or Reference | FORMULATION | $t_{50\%}$ (h) | $t_p$0.5 (h) |
|---|---|---|---|
| **Aminoglutethimdie** | **J** | 7.6 | 7.6 |
| | **E** | 4.6 | 5.1 |
| | **F** | 7.9 | 7.9 |
| ***Orimeten®*** | ***Marketed immediate release*** | <0.17 | <1 |
| | | | |
| **Cimetidine** | **J** | 5.7 | 6.0 |
| | **E** | 3.6 | 4.3 |
| | **F** | 7.8 | 7.8 |
| ***Acinil® 200mg*** | ***Marketed immediate release*** | <0.17 | < 1 |
| | | | |
| **Ketoconazole** | **J** | 1.9 | 3.1 |
| | **E** | 2.4 | 3.4 |
| | **F** | 4.2 | 4.6 |
| ***Nizoral® 200mg*** | ***Marketed immediate release*** | <0.17 | ~ 1 |
| | | | |
| **Metyrapone** | **J** | 0.7 | 1.8 |
| | **E** | 0.3 | 1.5 |
| | **F** | 0.4 | 1.5 |
| ***Metopiron® 250 mg*** | ***Marketed immediate release*** | <0.17 | <1 |

## Claims

1. A composition for controlled release of a cortisol antagonist comprising at least one release rate controlling substance together with said cortisol antagonist, wherein at least one of the release rate controlling substances is a fatty acid ester wherein the fatty acid component is a saturated or unsaturated fatty acid having between 6 and 26 carbon atoms and the composition is adapted for oral administration to a mammal.

2. A composition as claimed in claim 1 wherein the cortisol antagonist is selected from the group comprising sodium valporate, an ankephalin, an opioid, Clonidine, oxytocin, mifepristons, katoconazole, aminogluthetimide, metyrapone, etomidate, trilostane, mitotane, Trilostan, phenyltoin, procaine, vitamin C. a salicylate, cimetidine, lidocaine and analogues and functionally equivalent derivatives thereof.

**3.** A composition as claimed in claim 1 wherein the cortisol antagonist is a compound of formula (I)

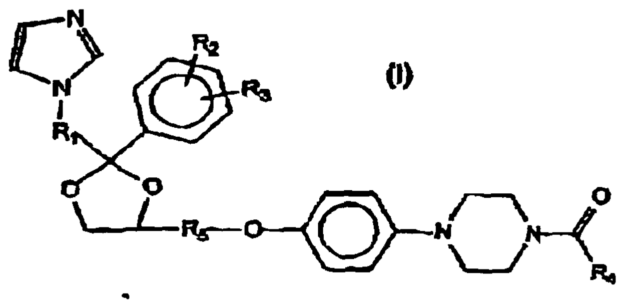

(I)

wherein $R_1$ and $R_5$ which may be the same or different represent a $C_{1-3}$ alkyl group, $R_2$ and $R_3$ which may be the same or different represent hydrogen, a halogen atom or a methyl group substituted by one or more halogen atoms and $R_4$ represents the group $(CH_2)_aCH_3$ wherein n may be 0, 1 or 2.

**4.** A composition as claimed in any preceding claim wherein the cortisol antagonist is ketoconazole.

**5.** A composition as claimed in claim 1 wherein the cortisol antagonist is a cortisol synthesis inhibitor.

**6.** A composition as claimed in any preceding claim which provides an *in vitro* release of cortisol antagonist which is sustained for at least 2 hours.

**7.** A composition as claimed in claim 6 which provides an in vitro release of cortisol antagonist which is sustained for at least 4 hours.

**8.** A composition as claimed in any preceding claim which provides an *in vivo* uptake of cortisol antagonist which is sustained for at least 2 hours.

**9.** A composition as claimed in claim 8 which provides an in vivo uptake of cortisol antagonist which is sustained for at least 4 hours.

**10.** A composition as claimed in claim 4 wherein the composition provides circulating plasma levels of ketoconazole in excess of 200 ng/ml for at least 4 hours.

**11.** A composition as claimed in any preceding claim wherein circulating plasma levels of cortisol antagonist do not reach pharmaceutically effective levels until more than 30 mins after administration of said composition to a patient.

**12.** A composition as claimed in any preceding claim wherein at least one of the release rate controlling substances is a solid, semi-solid, amorphous, liquid crystalline or liquid matrix, preferably a solid or semi-solid matrix, in which the cortisol antagonist is dispersed.

**13.** A composition as claimed in any preceding claim wherein the fatty acid component is selected from the group comprising palmitoleic acid, oleic acid, linoleic acid, linolenic acid and arachidonic acid.

**14.** A composition as claimed in any preceding claim wherein the fatty acid ester is formed between a polyhydric alcohol selected from the group comprising glycerol, 1,2-propanediol, 1,3-propanediol, diacylgalactosylglycerol, diacyldigalactosylglycerol, erythritol, xylitol, adonitol, arabitol, mannitol, and sorbitol and a fatty acid.

**15.** A composition as claimed in claims 1 to 13 wherein the fatty acid ester is formed between a hydroxycarboxylic acid selected from the group comprising malic acid, tartaric acid, citric acid, and lactic acid and a fatty acid.

**16.** A composition as claimed in claims 1 to 13 wherein the hydroxy-containing component of the fatty acid ester is a

saccharide selected from the group comprising glucose, mannose, fructose, threose, gulose, arabinose, ribose, erythrose, lyxose, galactose, sorbose, altrose, tallose, idose, rhamnose and allose or a glycerylphosphate derivative selected from the group comprising phosphatidic acid, phosphatidylserine, phosphatidylethanolamine, phosphatidylcholine, phosphatidylglycerol, phosphatidylinositole, and diphosphatidylglycerol.

**17.** A composition as claimed in any one of claims 1 to 12 wherein at least one of the release rate controlling substances is a fatty acid ester selected from the group comprising dioleyol phosphatidyl-cholin, dilauryl phosphatidylcholin, dimyristyl phosphatidylcholin, dipalmitoyl phosphatidylcholin, distearoyl phosphatidylcholin, dibehenoyl phosphatidyl-cholin, dimyristyl phosphatidylethanolamine, dipalmitoyl phosphatidylethanolsmine, dioleyl phosphatidylglycerol, dilauryl phosphatidylglycerol, dimyristoyl phosphatidylglycerol, dipalmitoyl phosphatidylglycerol, distearoyl phosphatidylglycerol, dipalmitoyl phosphatic acid and mixtures thereof.

**18.** A composition as claimed in any preceding claim wherein at least one of the release rate controlling substances is a mono- or di-glyceride.

**19.** A composition as claimed in claim 18 wherein at least one of the release rate controlling substances is selected from the group comprising glycerol dioleate, glyceryl monooleate and glyceryl monolinoleate.

**20.** A composition as claimed in any preceding claim wherein at least one of the release rate controlling substances is a polymer.

**21.** A composition as claimed in claim 20 wherein the polymer is selected from the group comprising alginates, polyacrylic acids (carbopols), chitosan, carboxyvinyl polymers, cellulose and cellulose derivatives such as hydroxypropyl methylcellulose, calcium or sodium carboxymethylcellulose, microcrystalline cellulose, powdered cellulose and starch and derivatives thereof.

**22.** A composition as claimed in any one of claims 1 to 12 which comprises, as release rate controlling substances a mixture of a fatty acid ester, oleic acid or sesame oil and/or a cellulose based polymer.

**23.** A composition as claimed in any preceding claim which is adapted for oral administration to a human.

**24.** A composition as claimed in any preceding claim which retains a substantially unitary form on administration and throughout the controlled release of the cortisol antagonist.

**25.** A method of making a composition for controlled release of a cortisol antagonist wherein said composition is adapted for oral administration, which method comprises mixing said cortisol antagonist with at least one release rate controlling substance, and wherein at least one of the release rate controlling substances is a fatty acid ester wherein the fatty acid component is a saturated or unsaturated fatty acid having between 6 and 26 carbon atoms.

**26.** A composition as claimed in any one of claims 1 to 24 for use in therapy.

**27.** A composition as claimed in any one of claims 1 to 24 for use in the treatment of diseases **characterised by** or associated with hypercortisolemia.

**28.** A composition as claimed in any one of claims 1 to 24 for use in human therapy.

**29.** A composition as claimed in any one of claims 1 to 24 for use in the treatment of metabolic syndrome or diabetes mellitis type II.

**30.** The use of a cortisol antagonist and a release rate controlling substance, wherein the release rate controlling substance is a fatty acid ester wherein the fatty acid component is a saturated or unsaturated fatty acid having between 6 and 26 carbon atoms in the manufacture of a medicament allowing controlled release of said cortisol antagonist for the treatment of metabolic syndrome or diabetes mellitis type II, wherein said medicament is adapted for oral administration.

## Patentansprüche

1. Zusammensetzung für eine kontrollierten Freisetzung eines Cortisolantagonisten, umfassend mindestens eine Substanz, welche die Freisetzungsrate kontrolliert, zusammen mit dem Cortisolantagonisten, worin mindestens eine der Substanzen, welche die Freisetzungsrate kontrollieren, ein Fettsäureester ist, worin der Fettsäurebestandteil eine gesättigte oder ungesättigte Fettsäure mit zwischen 6 und 26 Kohlenstoffatomen ist, und worin die Zusammensetzung für eine orale Verabreichung an einen Säuger geeignet ist.

2. Zusammensetzung nach Anspruch 1, worin der Cortisolantagonist ausgewählt ist aus der Gruppe umfassend Natriumvalporat, ein Enkephalin, ein Opioid, Clonidin, Oxytocin, Mifepriston, Ketoconazol, Aminogluthetimid, Metyrapon, Etomidat, Trilostane, Mitotan, Trilostan, Phenyltoin, Procain, Vitamin C, ein Salicylat, Cimetidin, Lidocain und Analoga und funktionell äquivalente Derivate davon.

3. Zusammensetzung nach Anspruch 1, worin der Cortisolantagonist eine Verbindung mit der Formel (I) ist

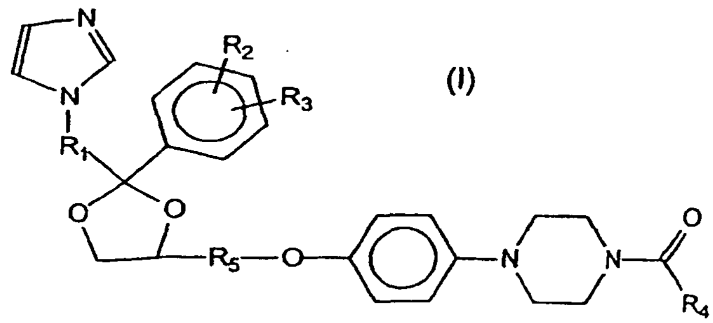

(I)

worin $R_1$ und $R_5$, welche gleich oder verschieden sein können, eine $C_{1-3}$-Alkylgruppe darstellen, $R_2$ und $R_3$, welche gleich oder verschieden sein können, Wasserstoff, ein Halogenatom oder eine Methylgruppe, welche mit einem oder mehreren Halogenatomen substituiert ist, darstellen und $R_4$ die Gruppe $(CH_2)_nCH_3$ darstellt, worin n 0, 1 oder 2 sein kann.

4. Zusammensetzung nach einem der vorhergehenden Ansprüche, worin der Cortisolantagonist Ketoconazol ist.

5. Zusammensetzung nach Anspruch 1, worin der Cortisolantagonist ein Inhibitor der Cortisolsynthese ist.

6. Zusammensetzung nach einem der vorhergehenden Ansprüche, welche *in vitro* eine Freisetzung eines Cortisolantagonisten ermöglicht, welche für mindestens 2 Stunden aufrechterhalten wird.

7. Zusammensetzung nach Anspruch 6, welche *in vitro* eine Freisetzung eines Cortisolantagonisten ermögücht, welche für mindestens 4 Stunden aufrechterhalten wird.

8. Zusammensetzung nach einem der vorhergehenden Ansprüche, welche *in vivo* eine Aufnahme eines Cortisolantagonisten ermöglicht, welche für mindestens 2 Stunden aufrechterhalten wird.

9. Zusammensetzung nach Anspruch 8, welche *in vivo* eine Aufnahme eines Cortisolantagonisten ermöglicht, welche für mindestens 4 Stunden aufrechterhalten wird.

10. Zusammensetzung nach Anspruch 4, worin die Zusammensetzung für mindestens 4 Stunden zirkulierende Ketoconazol-Plagmaspiegel von mehr als 200 ng/ml ermöglicht.

11. Zusammensetzung nach einem der vorhergehenden Ansprüche, worin die zirkulierenden Plasmaspiegel eines Cortisolantagonisten bis mehr als 30 Minuten nach Verabreichung der Zusammensetzung an einen Patienten keine pharmazeutisch wirksamen Spiegel erreichen.

12. Zusammensetzung nach einem der vorhergehenden Ansprüche, worin mindestens eine der Substanzen, welche die Freisetzungsrate kontrollieren, eine feste, halbfeste, amorphe, flüssigkristalline oder flüssige Matrix ist, bevorzugt eine feste oder halbfeste Matrix, in welcher der Cortisolantagonist verteilt ist.

**13.** Zusammensetzung nach einem der vorhergehenden Ansprüche, worin der Fettsäurebestandteil ausgewählt ist aus der Gruppe umfassend Palmitoleinsäure, Oleinsäure, Linolsäure, Linolensäure und Arachidonsäure.

**14.** Zusammensetzung nach einem der vorhergehenden Ansprüche, worin der Fettsäureester zwischen einem mehrwertigen Alkohol, welcher ausgewählt ist aus der Gruppe umfassend Glycerin, 1,2-Propandiol, 1,3-Propandiol, Diacylgalaktosylglycerin, Diacyldigalaktosylglycerin, Erythritol, Xylitol, Adonitol, Arabitol, Mannitol und Sorbitol, und einer Fettsäure gebildet ist.

**15.** Zusammensetzung nach einem der Ansprüche 1 bis 13, worin der Fettsäureester gebildet wird zwischen einer Hydroxycarbonsäure, welche ausgewählt ist aus der Gruppe umfassend Äpfelsäure, Weinsäure, Citronensäure und Milchsäure, und einer Fettsäure.

**16.** Zusammensetzung nach einem der Ansprüche 1 bis 13, worin der Hydroxyhaltige Bestandteil des Fettsäureesters ein Saccharid ist, welches ausgewählt ist aus der Gruppe umfassend Glukose, Mannose, Fructose, Threose, Gulose, Arabinose, Ribose, Erythrose, Lyxose, Galaktose, Sorbose, Altrose, Tallose, Idose, Rhamnose und Allose, oder ein Gycerylphosphat-Derivat, welches ausgewählt ist aus der Gruppe umfassend Phosphatidsäure, Phosphatidylserin, Phosphatidylethanolamin, Phosphatidylcholin, Phosphatidylglycerin, Phosphatidylinositol und Diphosphatidylglycerin.

**17.** Zusammensetzung nach einem der Ansprüche 1 bis 12, worin mindestens eine der Substanzen, welche die Freisetzungsrate kontrollieren, ein Fettsäureester ist, welcher ausgewählt ist aus der Gruppe umfassend Dioleyolphosphatidylcholin, Dilaurylphosphatidylcholin, Dimyristylphosphatidylcholin, Dipalmitoylphosphatidylcholin, Distearorylphosphatidylcholin, Dibehenoylphosphatidylcholin, Dimyristylphosphatidylethanolamin, Dipalmitoylphosphatidylethanolamin, Dioleylphosphatidylglycerin, Dilaurylphosphatidylglycerin, Dimyristoylphosphatidylglycerin, Dipalmitoylphosphatidylglycerin, Distearoylphosphatidylglycerin, Dipalmitoyl-phosphatische Säure und Gemischen davon.

**18.** Zusammensetzung nach einem der vorhergehenden Ansprüche, worin mindestens eine der Substanzen, welche die Freisetzungsrate kontrollieren, ein Mono- oder ein Diglycerid ist.

**19.** Zusammensetzung nach Anspruch 18, worin mindestens eine der Substanzen, welche die Freisetzungsrate kontrollieren, ausgewählt ist aus der Gruppe umfassend Glycerindioleat, Glycerylmonooleat und Glycerylmonolinoleat.

**20.** Zusammensetzung nach einem der vorhergehenden Ansprüche, worin mindestens eine der Substanzen, welche die Freisetzungsrate kontrollieren, ein Polymer ist.

**21.** Zusammensetzung nach Anspruch 20, worin das Polymer ausgewählt ist aus der Gruppe umfassend Alginate, Polyacrylsäuren (Carbopole), Chitosan, Carboxyvinylpolymere, Cellulose und Cellulosederivate wie etwa Hydroxypropylmethylcellulose, Calcium- oder Natriumcarboxymethylcellulose, mikrokristalline Cellulose, pulverförmige Cellulose und Stärke und Derivate davon.

**22.** Zusammensetzung nach einem der Ansprüche 1 bis 12, welche als Substanz, welche die Freisetzungsrate kontrolliert, ein Gemisch eines Fettsäureesters, Ölsäure oder Sesamöl und/oder ein auf Cellulose basierendes Polymer umfasst.

**23.** Zusammensetzung nach einem der vorhergehenden Ansprüche, welche für eine orale Verabreichung an einen Menschen geeignet ist.

**24.** Zusammensetzung nach einem der vorhergehenden Ansprüchen, welche bei der Verabreichung und während der kontrollierten Freisetzung des Cortisolantagonisten eine im Wesentlichen einheitliche Form beibehält.

**25.** Verfahren zur Herstellung einer Zusammensetzung für eine kontrollierte Freisetzung eines Cortisolantagonisten, worin die Zusammensetzung für eine orale Verabreichung geeignet ist, worin das Verfahren Mischen des Cortisolantagonisten mit mindestens einer Substanz, welche die Freisetzungsrate kontrolliert, umfasst, und worin mindestens eine der Substanzen, welche die Freisetzungsrate kontrollieren, ein Fettsäureester ist, worin der Fettsäurebestandteil eine gesättigte oder ungesättigte Fettsäure mit zwischen 6 und 26 Kohlenstoffatomen ist.

**26.** Zusammensetzung nach einem der Ansprüche 1 bis 24 zur Verwendung bei einer Therapie.

**27.** Zusammensetzung nach einem der Ansprüche 1 bis 24 zur Verwendung bei der Behandlung von Erkrankungen, welche durch eine Hyperchortisolämie **gekennzeichnet** sind oder mit einer Hyperchortisolämie verbunden sind.

**28.** Zusammensetzung nach einem der Ansprüche 1 bis 24 zur Verwendung bei einer Humantherapie.

**29.** Zusammensetzung nach einem der Ansprüche 1 bis 24 zur Verwendung bei der Behandlung eines Stoffwechselsyndroms oder von Diabetes mellitus Typ II.

**30.** Verwendung eines Cortisolantagonisten und einer Substanz, welche die Freisetzungsrate kontrolliert, worin die Substanz, welche die Freisetzungsrate kontrolliert, ein Fettsäureester ist, worin der Fettsäurebestandteil eine gesättigte oder ungesättigte Fettsäure mit zwischen 6 und 26 Kohlenstoffatomen ist, bei der Herstellung eines Arzneimittels, welches eine kontrollierte Freisetzung des Cortisolantagonisten ermöglicht, zur Behandlung eines Stoffwechselsyndroms oder von Diabetes mellitus Typ II, worin das Arzneimittel für eine orale Verabreichung geeignet ist.

**Revendications**

**1.** Composition de libération réglée d'un antagoniste de cortisol comprenant au moins une substance réglant la vitesse de libération, ensemble avec l'antagoniste de cortisol, au moins l'une des substances réglant la vitesse de libération étant un ester d'acide gras, dans lequel le constituant acide gras est un acide gras saturé ou insaturé ayant entre 6 et 26 atomes de carbone et la composition est adaptée pour une administration orale à un mammifère.

**2.** Composition suivant la revendication 1, dans laquelle l'antagoniste de cortisol est choisi dans le groupe comprenant le valporate de sodium, une encéphaline, un opioïde, la clonidine, l'oxytocine, la mifépristone, le kétoconazole, l'aminogluthétimide, la métyrapone, l'étomidate, le trilostane, le mitotane, le trilostane, la phényltoïne, la procaïne, la vitamine C, un salicylate, la cimétidine, la lidocaïne et leurs analogues et dérivés équivalents fonctionnellement.

**3.** Composition suivant la revendication 1, dans laquelle l'antagoniste de cortisol est un composé de formule (I)

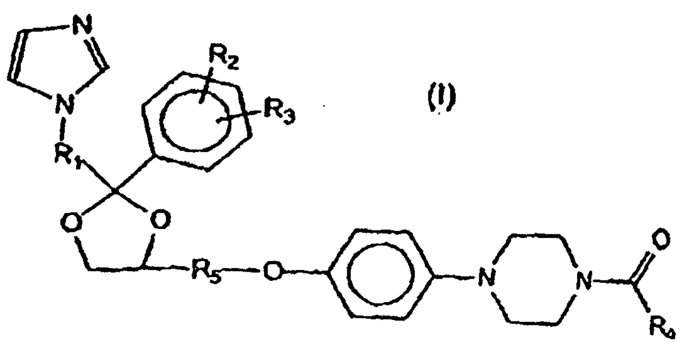

(I)

dans laquelle $R_1$ et $R_5$, qui peuvent être identiques ou différents, représentent un groupe alcoyle ayant de 1 à 3 atomes de carbone, $R_2$ et $R_3$, qui peuvent être identiques ou différents, représentent l'hydrogène, un atome d'halogène ou un groupe méthyle substitué par un ou par plusieurs atomes d'halogène et $R_4$ représente le groupe $(CH_2)nCH_3$ dans lequel n peut être 0, 1 ou 2.

**4.** Composition suivant l'une quelconque des revendications précédentes, dans laquelle l'antagoniste de cortisol est le kétoconazole.

**5.** Composition suivant la revendication 1, dans laquelle l'antagoniste de cortisol est un inhibiteur de la synthèse du cortisol.

**6.** Composition suivant l'une quelconque des revendications précédentes, qui donne une libération in vitro d'un antagoniste de cortisol qui se maintient pendant au moins 2 heures.

**7.** Composition suivant la revendication 6, qui donne une libération in vitro d'un antagoniste de cortisol qui se maintient pendant au moins 4 heures.

**8.** Composition suivant l'une quelconque des revendications précédentes, qui donne une absorption in vivo d'un antagoniste de cortisol qui se maintient pendant au moins 2 heures.

**9.** Composition suivant la revendication 8, qui donne une absorption in vivo d'un antagoniste de cortisol qui se maintient pendant au moins 4 heures.

**10.** Composition suivant la revendication 4, dans laquelle la composition donne des niveaux plasmatiques de circulation de kétoconazole dépassant 200 ng/ml pendant au moins 4 heures.

**11.** Composition suivant l'une quelconque des revendications précédentes, dans laquelle les niveaux plasmatiques de circulation d'antagoniste de cortisol n'atteignent pas des niveaux efficaces pharmaceutiquement jusqu'à plus de 30 minutes après l'administration de la composition à un patient.

**12.** Composition suivant l'une quelconque des revendications précédentes, dans laquelle au moins l'une des substances réglant la vitesse de libération est une matrice solide amorphe de cristal liquide, de préférence une matrice solide ou semi-solide dans laquelle l'antagoniste de cortisol est dispersé.

**13.** Composition suivant l'une quelconque des revendications précédentes, dans laquelle le constituant d'acide gras est choisi dans le groupe comprenant de l'acide palmitoléique, de l'acide oléique, de l'acide linoléique, de l'acide linolénique et de l'acide arachidonique.

**14.** Composition suivant l'une quelconque des revendications précédentes, dans laquelle l'ester d'acide gras est formé entre un polyalcool choisi dans le groupe comprenant le glycérol, le 1,2-propanediol, le 1,3-propanédiol, le diacylgalactosylglycérol, le diacyldigalactosylglycérol, l'érythritol, le xylitol, l'adonitol, l'arabitol, le mannitol et le sorbitol, et un acide gras.

**15.** Composition suivant l'une quelconque des revendications 1 à13, dans laquelle l'ester d'acide gras est formé entre un acide hydroxycarboxylique choisi dans le groupe comprenant l'acide malique, l'acide tartrique, l'acide citrique et l'acide lactique et un acide gras.

**16.** Composition suivant l'une des revendications 1 à 13, dans laquelle le constituant contenant un hydroxy de l'ester d'acide gras est un saccharide choisi dans le groupe comprenant le glucose, le mannose, le fructose, le thréose, le gulose, l'arabinose, le ribose, l'érythrose, le lyxose, le galactose, le sorbose, l'altrose, le tallose, l'idose, le rhamnose et l'allose ou un dérivé de glycérylphosphate choisi dans le groupe comprenant l'acide phosphatidique, la phosphatidylsérine, la phosphatidyléthanolamine, la phosphatidylcholine, le phosphatidylglycérol, le phosphatidylinositole et le diphosphatidylglycérol.

**17.** Composition suivant l'une quelconque des revendications 1 à 12, dans laquelle au moins l'une des substances réglant la vitesse de libération est un ester d'acide gras choisi dans le groupe comprenant la dioléyol phosphatidylcholine, la dilauryl phosphatidylcholine, la dimyristyl phosphatidylcholine, la dipalmitoyl phosphatidylcholine, la distéaroyl phosphatidylcholine, la dibéhénoyl phosphatidylcholine, la dimyristyl phosphatidyléthanolamine, la dipalmitoyl phosphatidyléthanolamine, le dioléyl phosphatidylglycérol, le distéaroyl phosphatidylglycérol, l'acide dipalmitoyl phosphatique et leurs mélanges.

**18.** Composition suivant l'une quelconque des revendications précédentes, dans laquelle au moins l'une des substances réglant la vitesse de libération est un monoglycéride ou un diglycéride.

**19.** Composition suivant la revendication 18, dans laquelle au moins l'une des substances réglant la vitesse de libération est choisie dans le groupe comprenant le dioléate de glycéryle, le monooléate de glycéryle et le monolinoléate de glycéryle.

**20.** Composition suivant l'une quelconque des revendications précédentes, dans laquelle au moins l'une des substances réglant la vitesse de libération est un polymère.

**21.** Composition suivant la revendication 20, dans laquelle le polymère est choisi dans le groupe comprenant des alginates, des poly(acides acryliques) (carbopols), du chitosane, des polymères de carboxyvinyl, de la cellulose et des dérivés de cellulose, tels que l'hydroxypropylméthylcellulose, la carboxyméthylcellulose de calcium ou la carboxyméthylcellulose de sodium, de la cellulose microcristalline, de la cellulose en poudre et de l'amidon, et leurs dérivés.

**22.** Composition suivant l'une quelconque des revendications 1 à 12, qui comprend comme substance réglant la vitesse de libération un mélange d'ester d'acide gras, d'acide oléique ou d'huile de sésame et/ou un polymère à base de cellulose.

**23.** Composition suivant l'une quelconque des revendications précédentes, qui est adaptée pour une administration orale à l'homme.

**24.** Composition suivant l'une quelconque des revendications précédentes, qui conserve une forme sensiblement unitaire après administration et pendant toute la libération réglée de l'antagoniste de cortisol.

**25.** Procédé de préparation d'une composition de libération réglée d'un antagoniste de cortisol dans lequel la composition est adaptée pour une administration orale, le procédé comprenant le mélange de l'antagoniste de cortisol a au moins une substance réglant la vitesse de libération et dans lequel au moins l'une des substances réglant la vitesse de libération est un ester d'acide gras, le constituant acide gras étant un acide gras saturé ou insaturé ayant entre 6 et 26 atomes de carbone.

**26.** Composition suivant l'une quelconque des revendications 1 à 24, pour utilisation en thérapeutique.

**27.** Composition suivant l'une quelconque des revendications 1 à 24, pour utilisation dans le traitement de maladies **caractérisées** ou associées à de l'hypercortisolémie.

**28.** Composition suivant l'une quelconque des revendications 1 à 24, pour utilisation en thérapeutique humaine.

**29.** Composition suivant l'une quelconque des revendications 1 à 24, pour utilisation dans le traitement de syndrome métabolique ou du diabète mellitis de type II.

**30.** Utilisation d'un antagoniste de cortisol et d'une substance réglant la vitesse de libération, la substance réglant la vitesse de libération étant un ester d'acide gras dans lequel le constituant acide gras est un acide gras saturé ou insaturé ayant entre 6 et 26 atomes de carbone dans la fabrication d'un médicament permettant une libération réglée de l'antagoniste de cortisol pour le traitement de syndrome métabolique ou du diabète mellitis de type II, le médicament étant adapté à une administration orale.

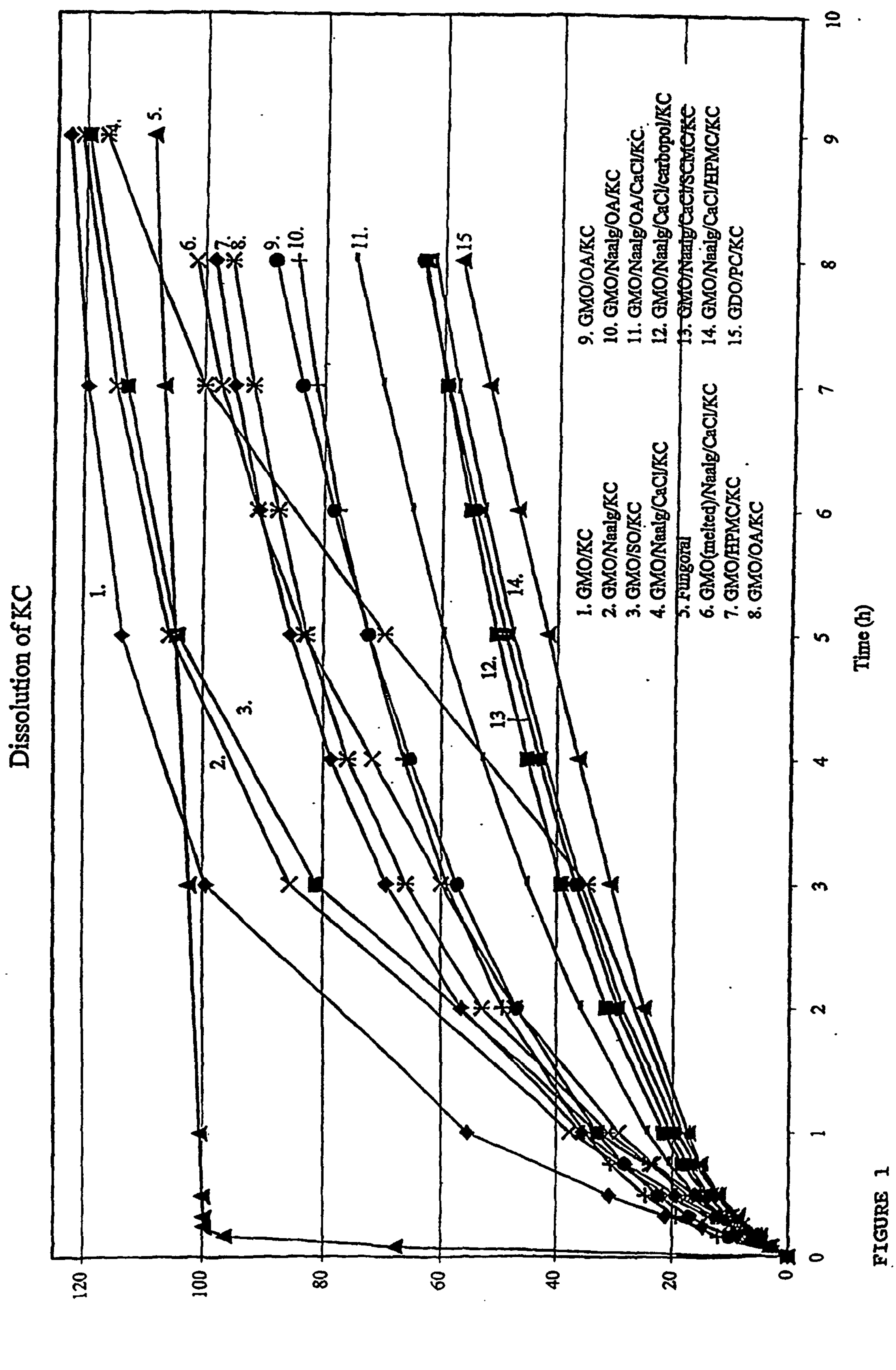

Dissolution of KC
Time (h)
1. GMO/KC
2. GMO/Naalg/KC
3. GMO/SO/KC
4. GMO/Naalg/CaCl/KC
5. Fungoral
6. GMO(melted)/Naalg/CaCl/KC
7. GMO/HPMC/KC
8. GMO/OA/KC
9. GMO/OA/KC
10. GMO/Naalg/OA/KC
11. GMO/Naalg/OA/CaCl/KC
12. GMO/Naalg/CaCl/carbopol/KC
13. GMO/Naalg/CaCl/SCMC/KC
14. GMO/Naalg/CaCl/HPMC/KC
15. GDO/PC/KC


FIGURE 1

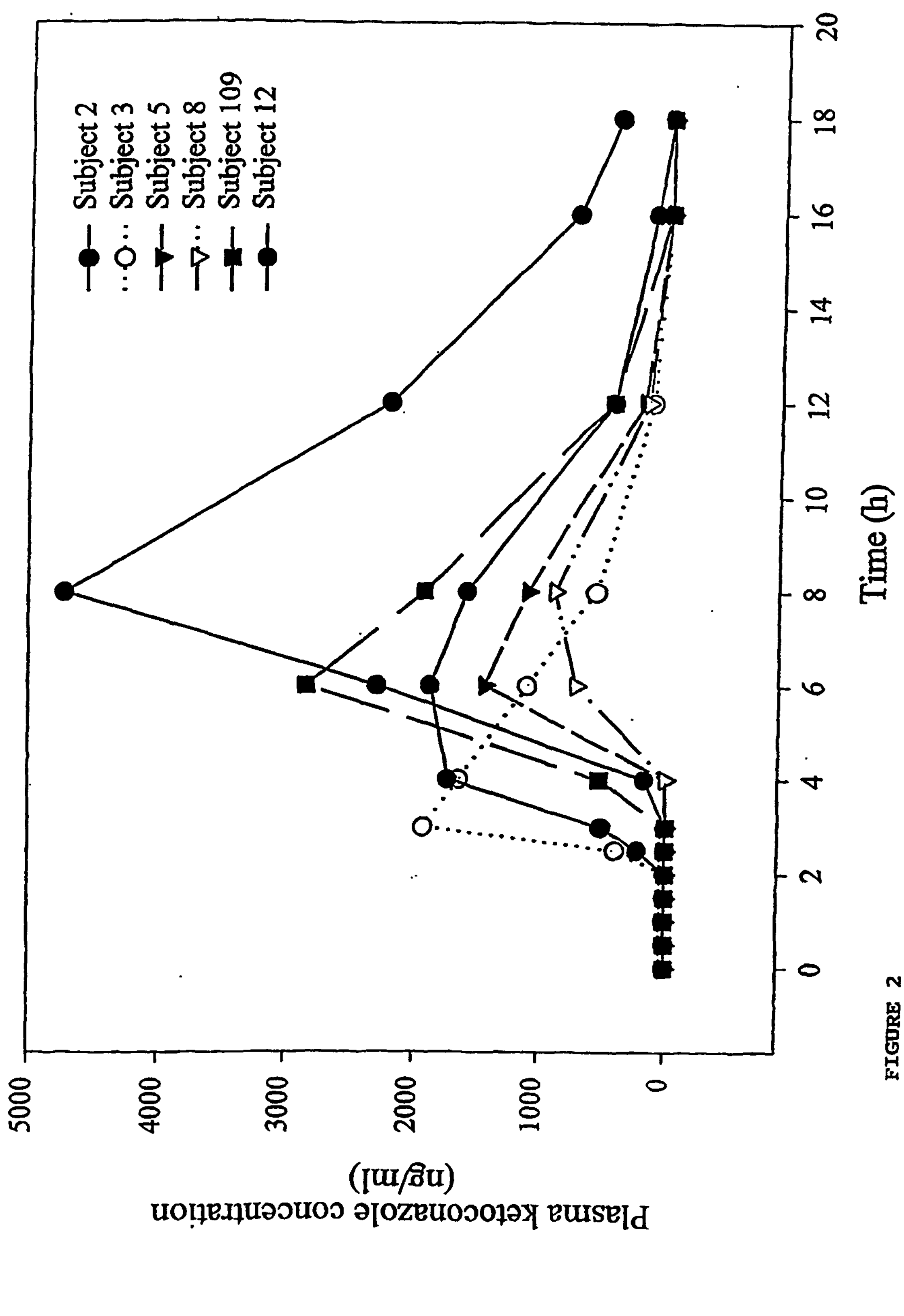
Plasma ketoconazole concentration (ng/ml)
5000
4000
3000
2000
1000
0
0
2
4
6
8
10
12
14
16
18
20
Time (h)
Subject 2
Subject 3
Subject 5
Subject 8
Subject 109
Subject 12

FIGURE 2

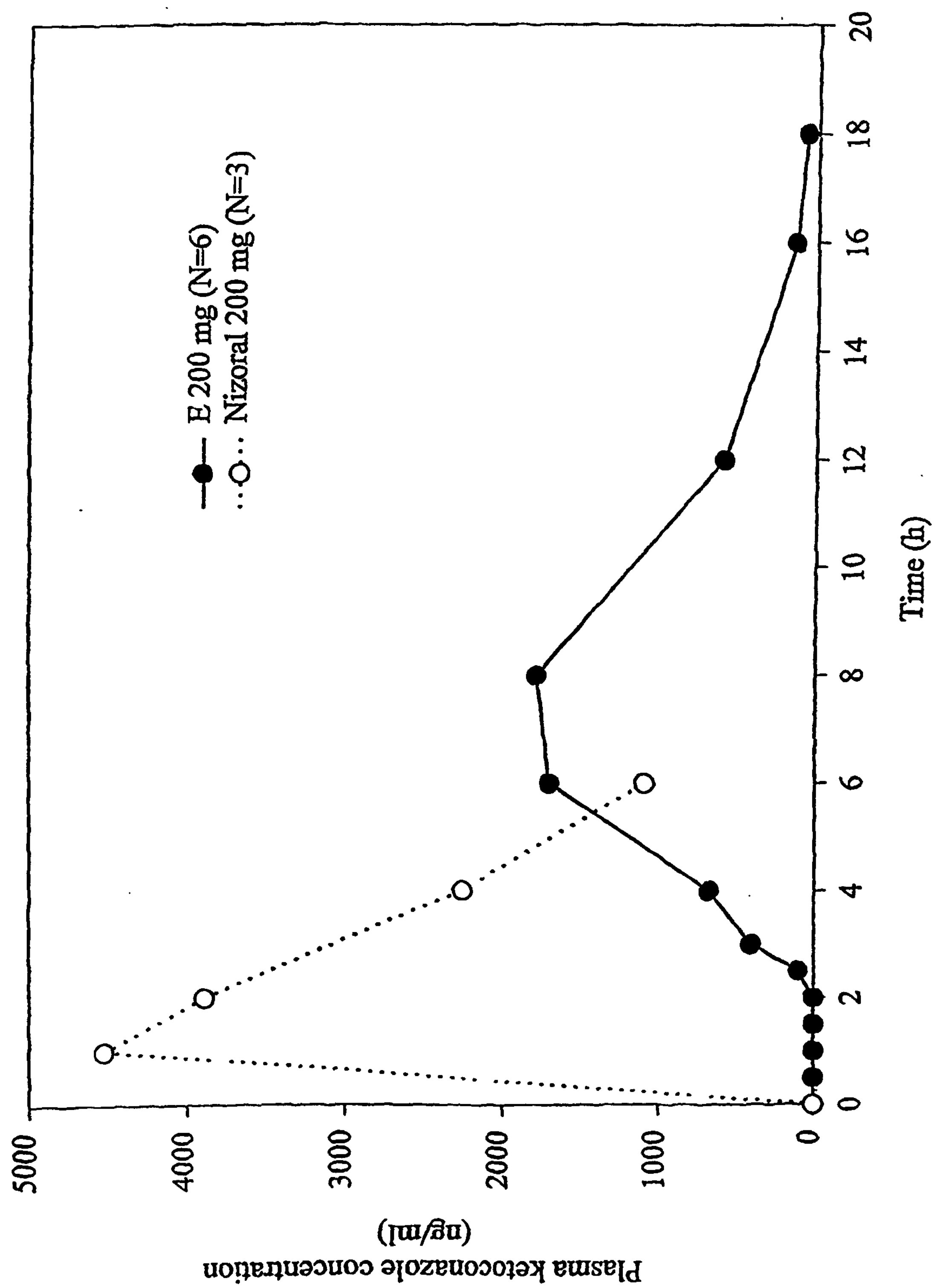
Plasma ketoconazole concentration (ng/ml)
5000
4000
3000
2000
1000
0
0
2
4
6
8
10
12
14
16
18
20
Time (h)
E 200 mg (N=6)
Nizoral 200 mg (N=3)

FIGURE 3

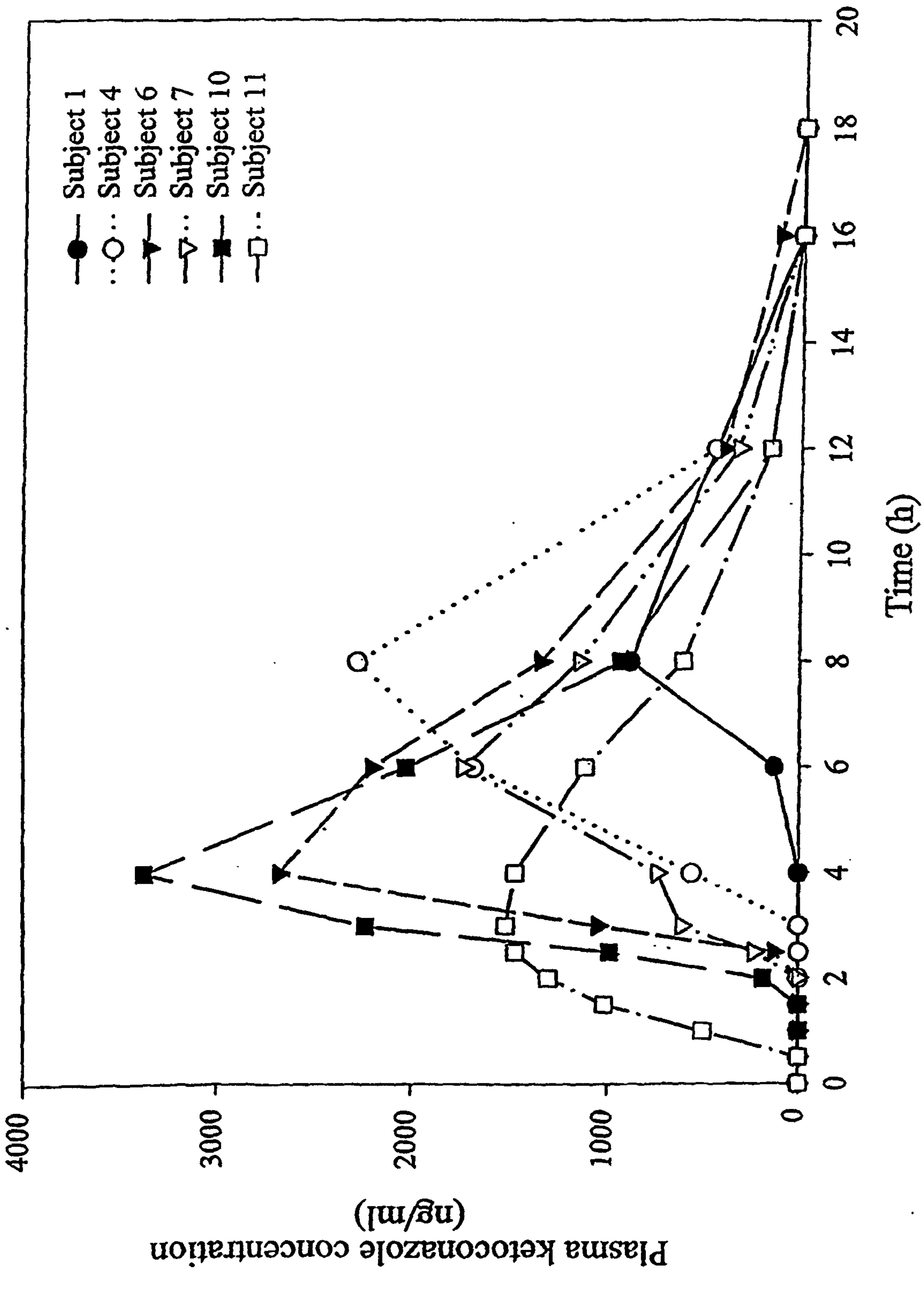
Subject 1
Subject 4
Subject 6
Subject 7
Subject 10
Subject 11
Plasma ketoconazole concentration (ng/ml)
0
1000
2000
3000
4000
Time (h)
0
2
4
6
8
10
12
14
16
18
20

FIGURE 4

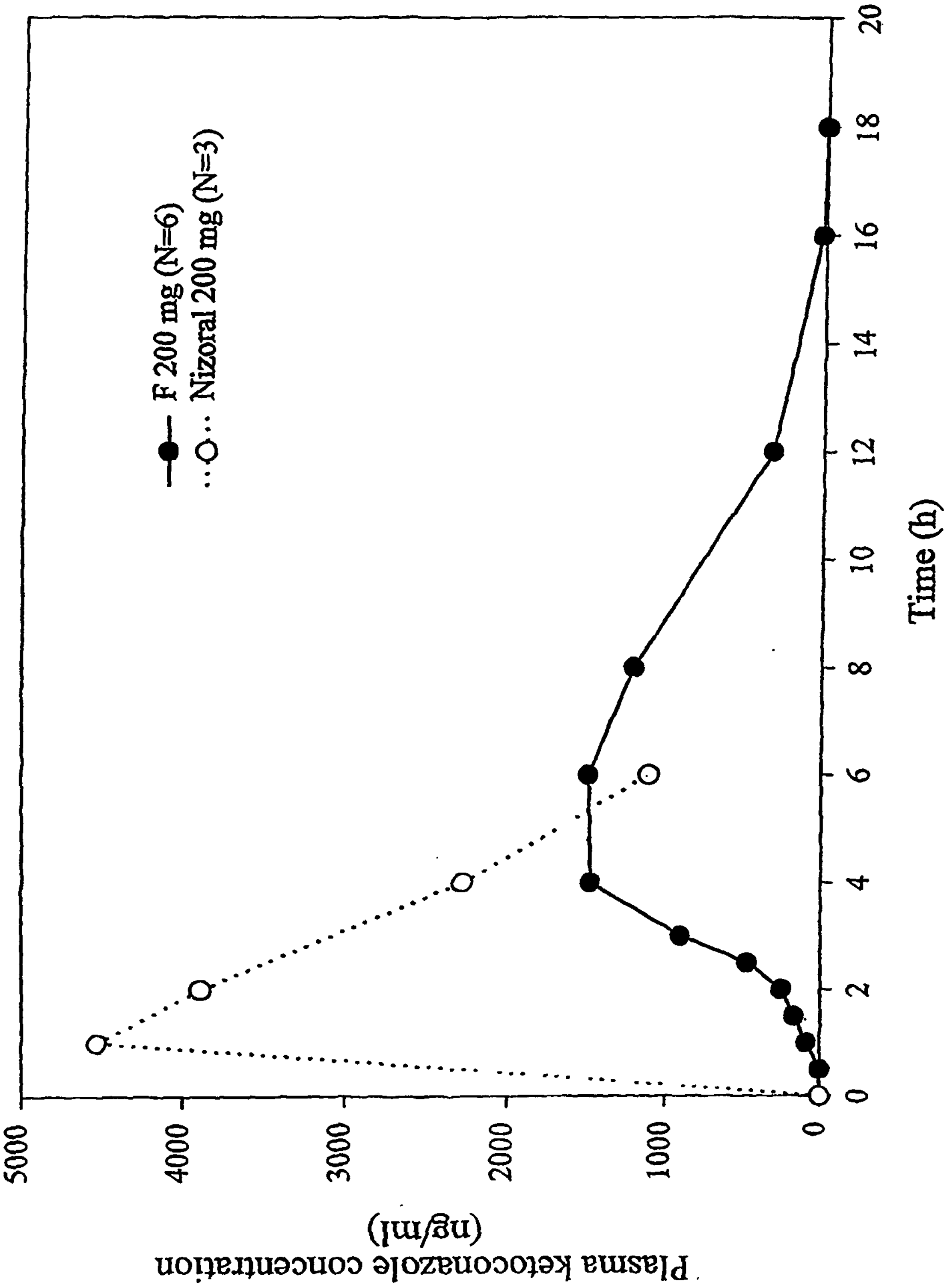
F 200 mg (N=6)
Nizoral 200 mg (N=3)
Plasma ketoconazole concentration (ng/ml)
0
1000
2000
3000
4000
5000
Time (h)
0
2
4
6
8
10
12
14
16
18
20

FIGURE 5

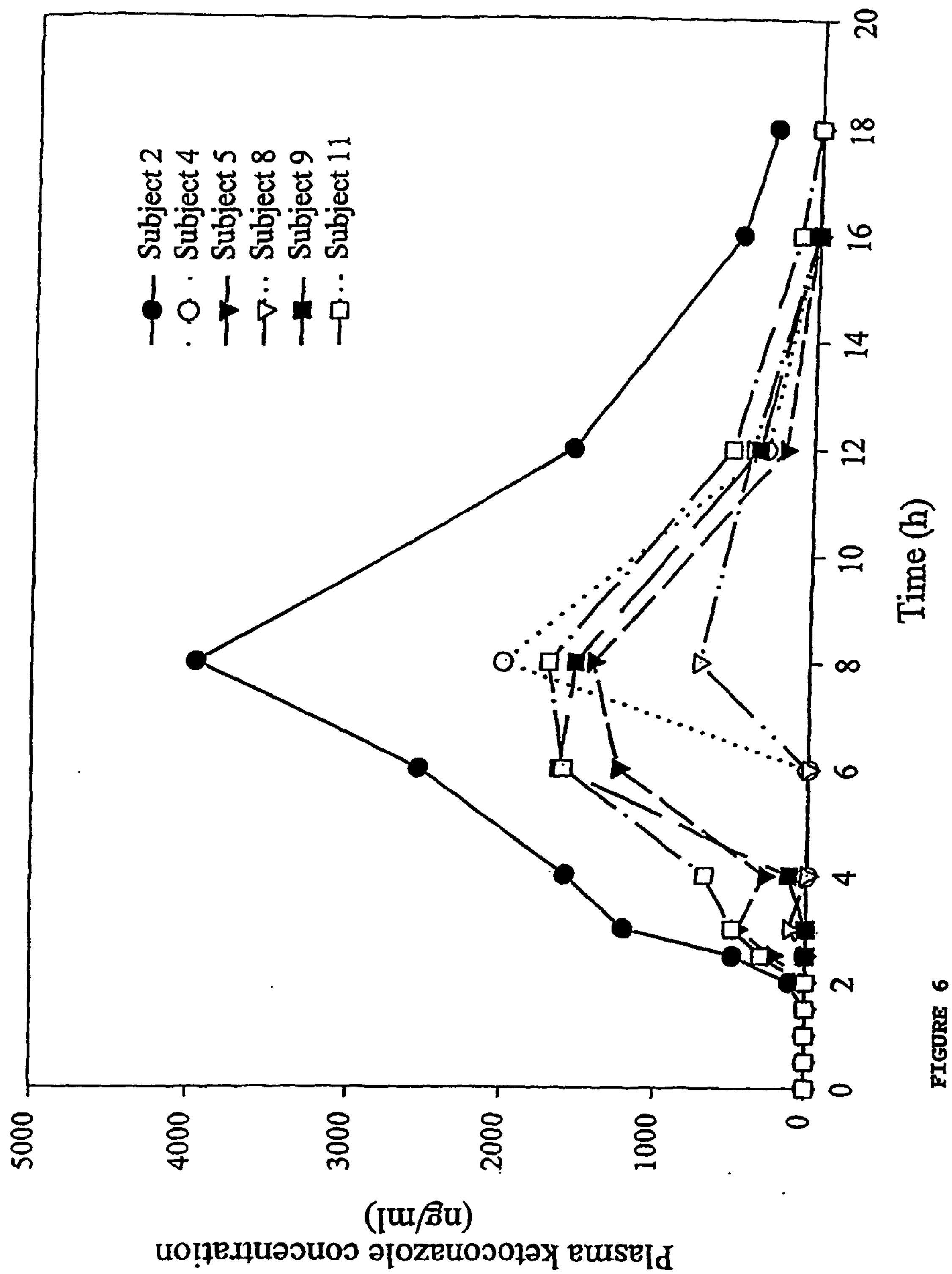
Plasma ketoconazole concentration (ng/ml)
5000
4000
3000
2000
1000
0
0
2
4
6
8
10
12
14
16
18
20
Time (h)
Subject 2
Subject 4
Subject 5
Subject 8
Subject 9
Subject 11

FIGURE 6

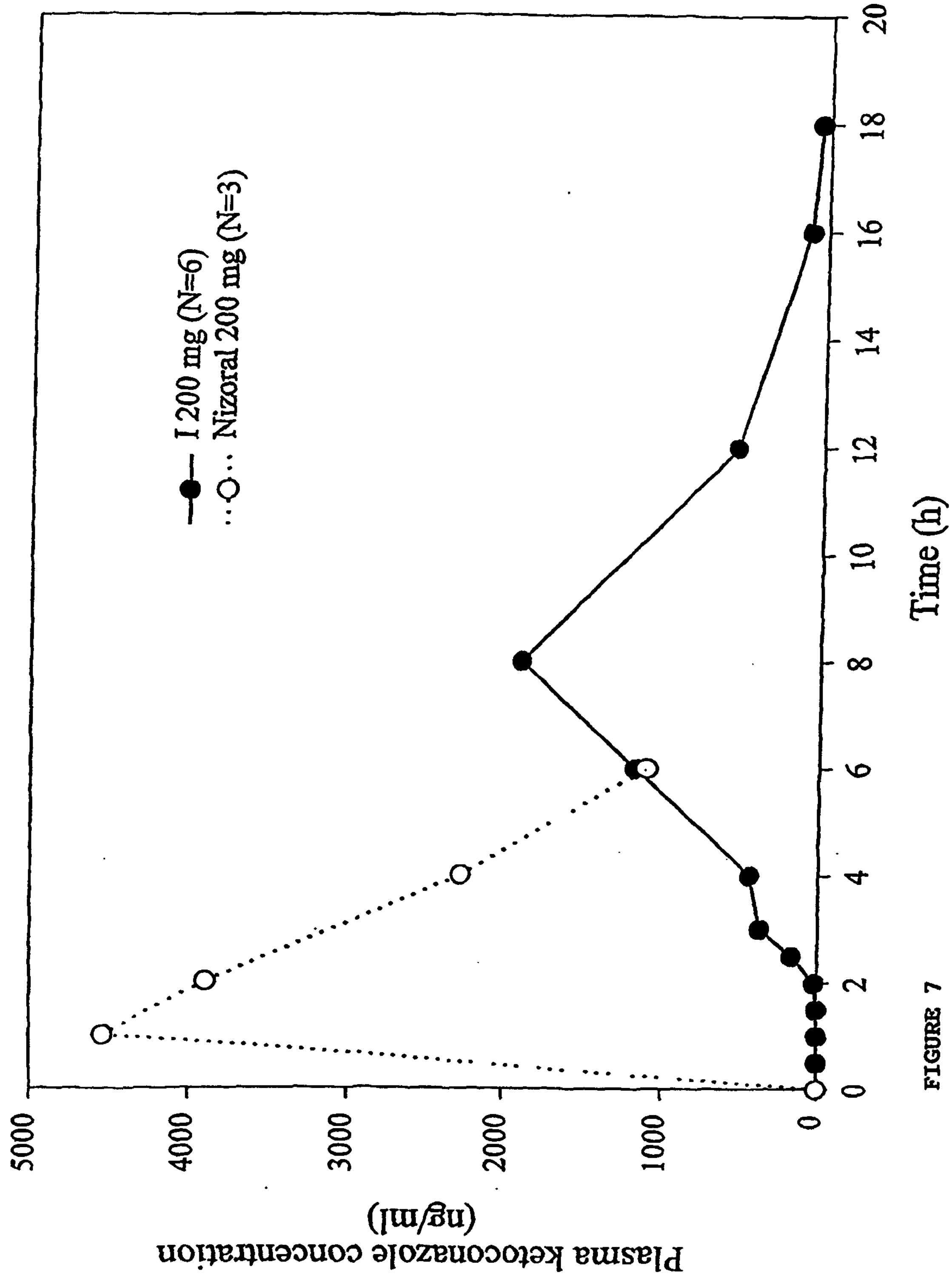
I 200 mg (N=6)
Nizoral 200 mg (N=3)
Plasma ketoconazole concentration (ng/ml)
0
1000
2000
3000
4000
5000
Time (h)
0
2
4
6
8
10
12
14
16
18
20

FIGURE 7

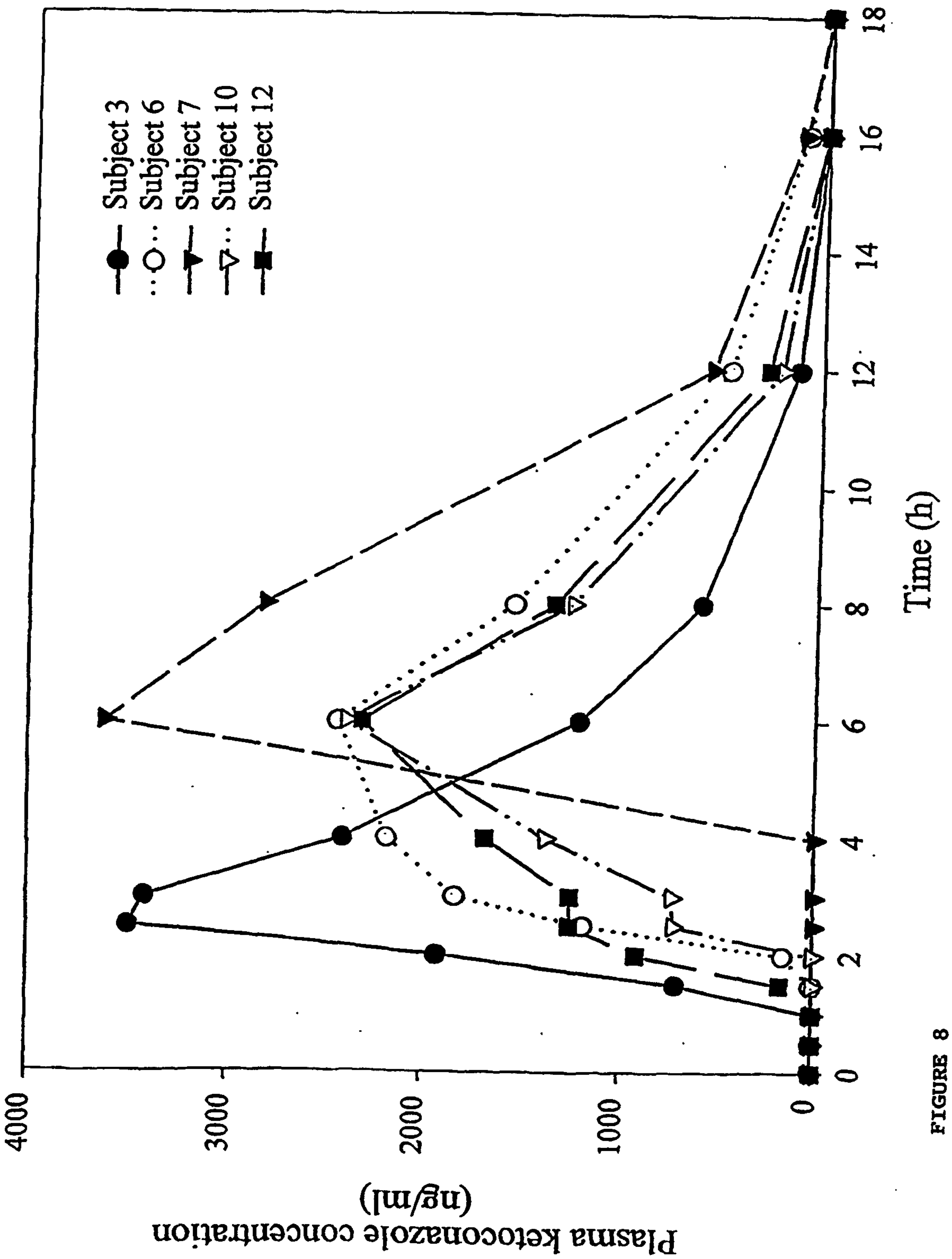
Subject 3
Subject 6
Subject 7
Subject 10
Subject 12
Plasma ketoconazole concentration (ng/ml)
4000
3000
2000
1000
0
0
2
4
6
8
10
12
14
16
18
Time (h)

FIGURE 8

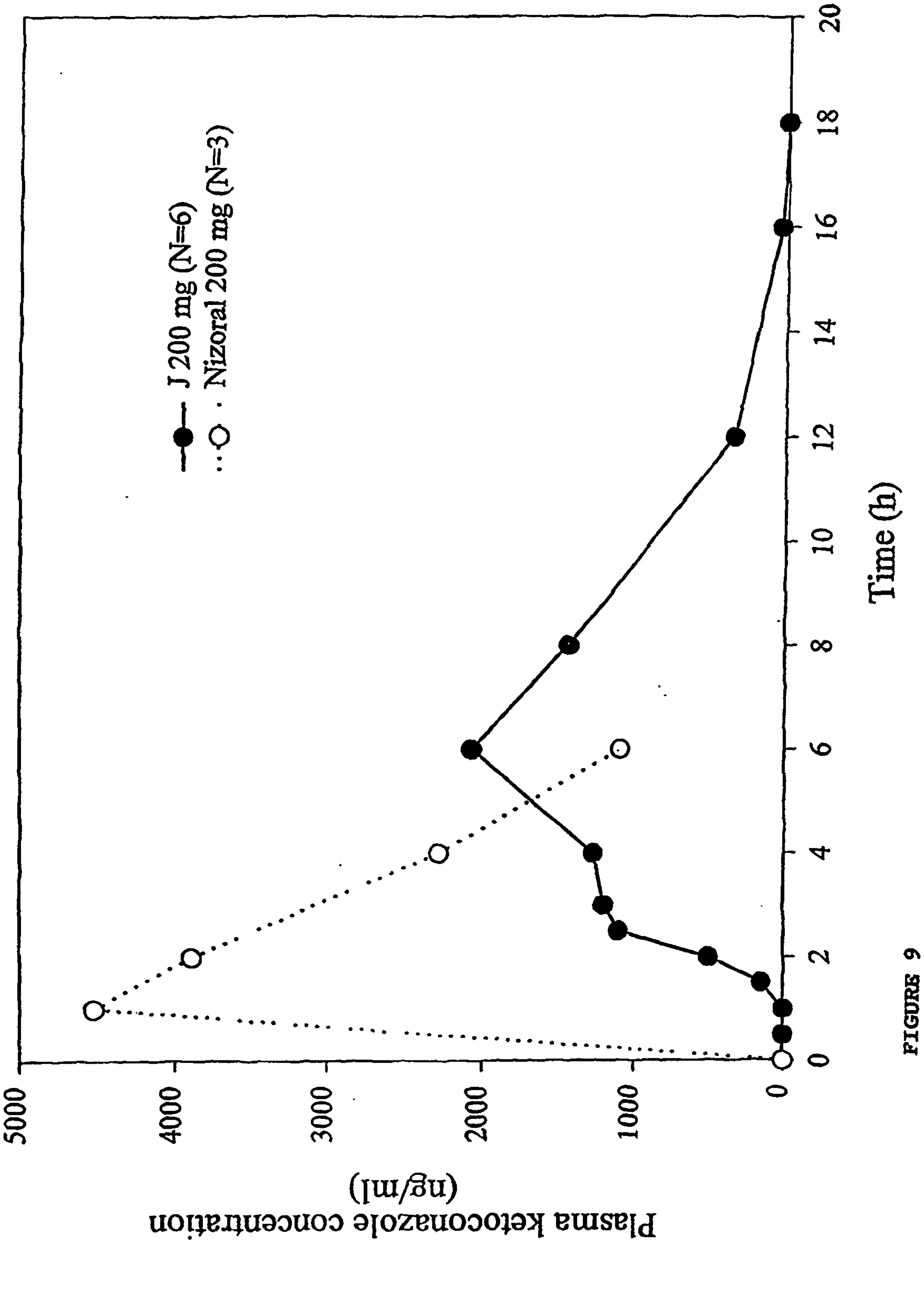
J 200 mg (N=6)
Nizoral 200 mg (N=3)
Plasma ketoconazole concentration (ng/ml)
Time (h)
0
1000
2000
3000
4000
5000
0
2
4
6
8
10
12
14
16
18
20

FIGURE 9

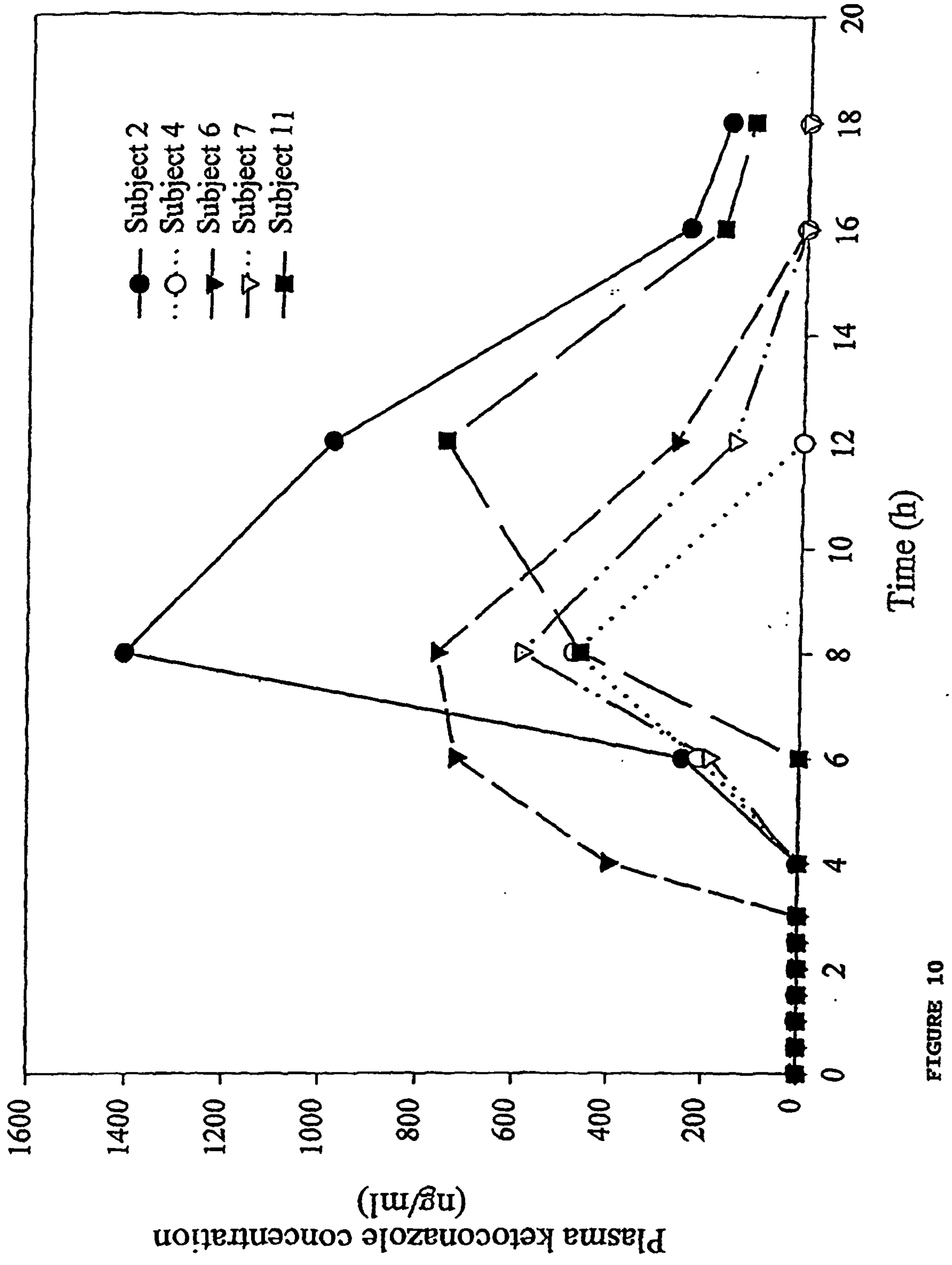
Plasma ketoconazole concentration (ng/ml)
0
200
400
600
800
1000
1200
1400
1600
Time (h)
0
2
4
6
8
10
12
14
16
18
20
Subject 2
Subject 4
Subject 6
Subject 7
Subject 11

FIGURE 10

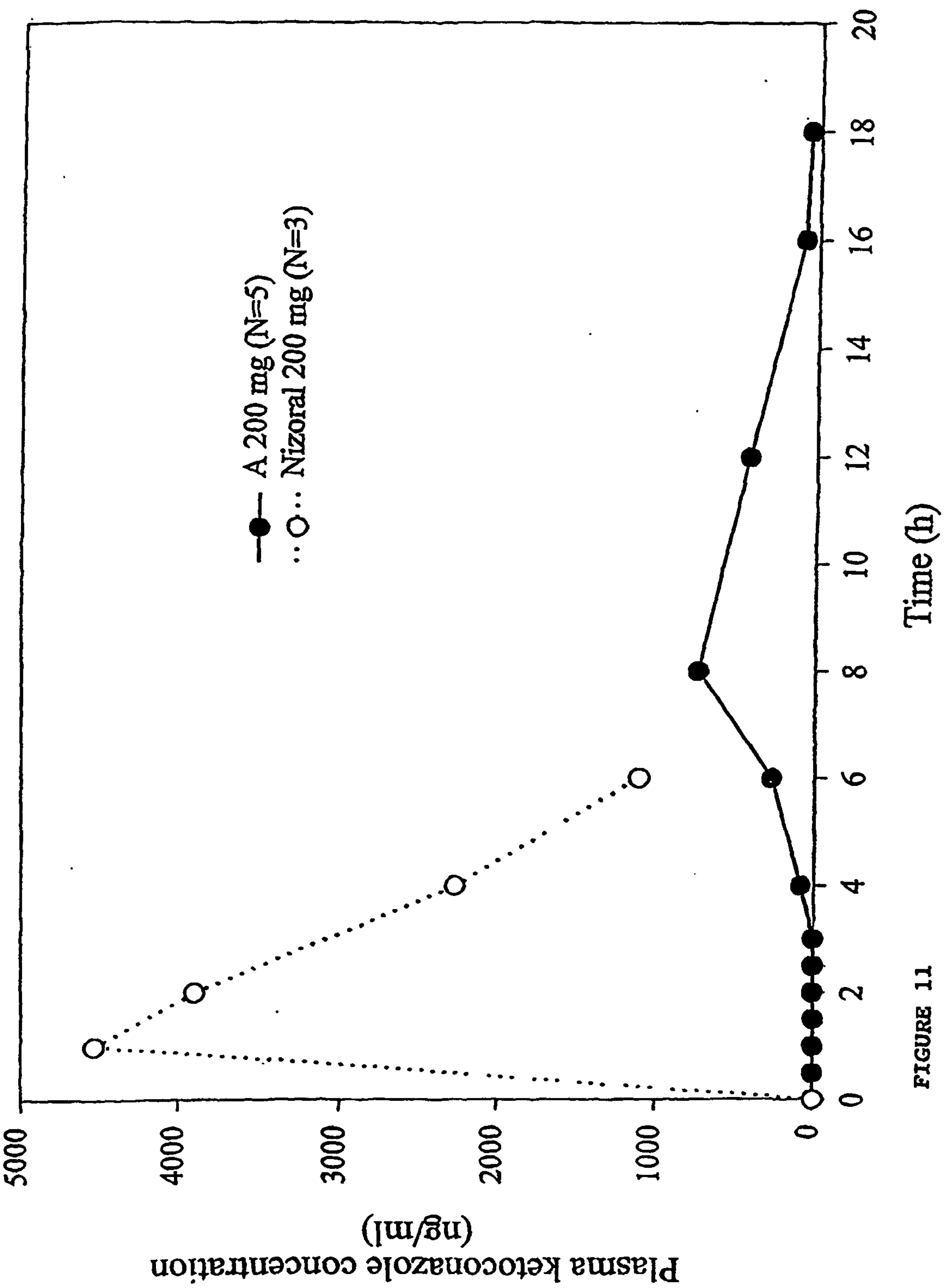
A 200 mg (N=5)
Nizoral 200 mg (N=3)
Plasma ketoconazole concentration (ng/ml)
Time (h)
0
1000
2000
3000
4000
5000
0
2
4
6
8
10
12
14
16
18
20

FIGURE 11

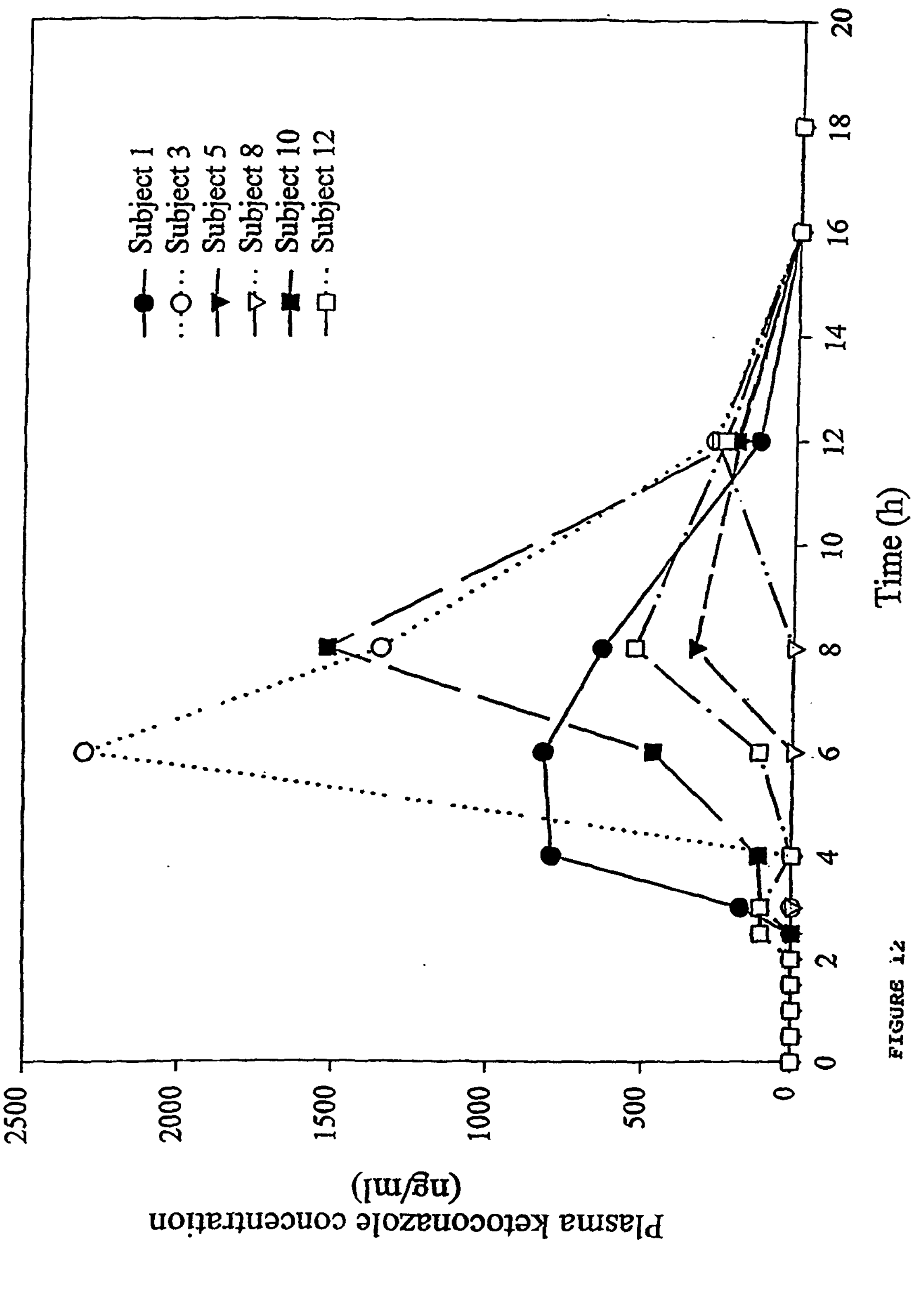
Plasma ketoconazole concentration (ng/ml)
2500
2000
1500
1000
500
0
Time (h)
0
2
4
6
8
10
12
14
16
18
20
Subject 1
Subject 3
Subject 5
Subject 8
Subject 10
Subject 12

FIGURE 12

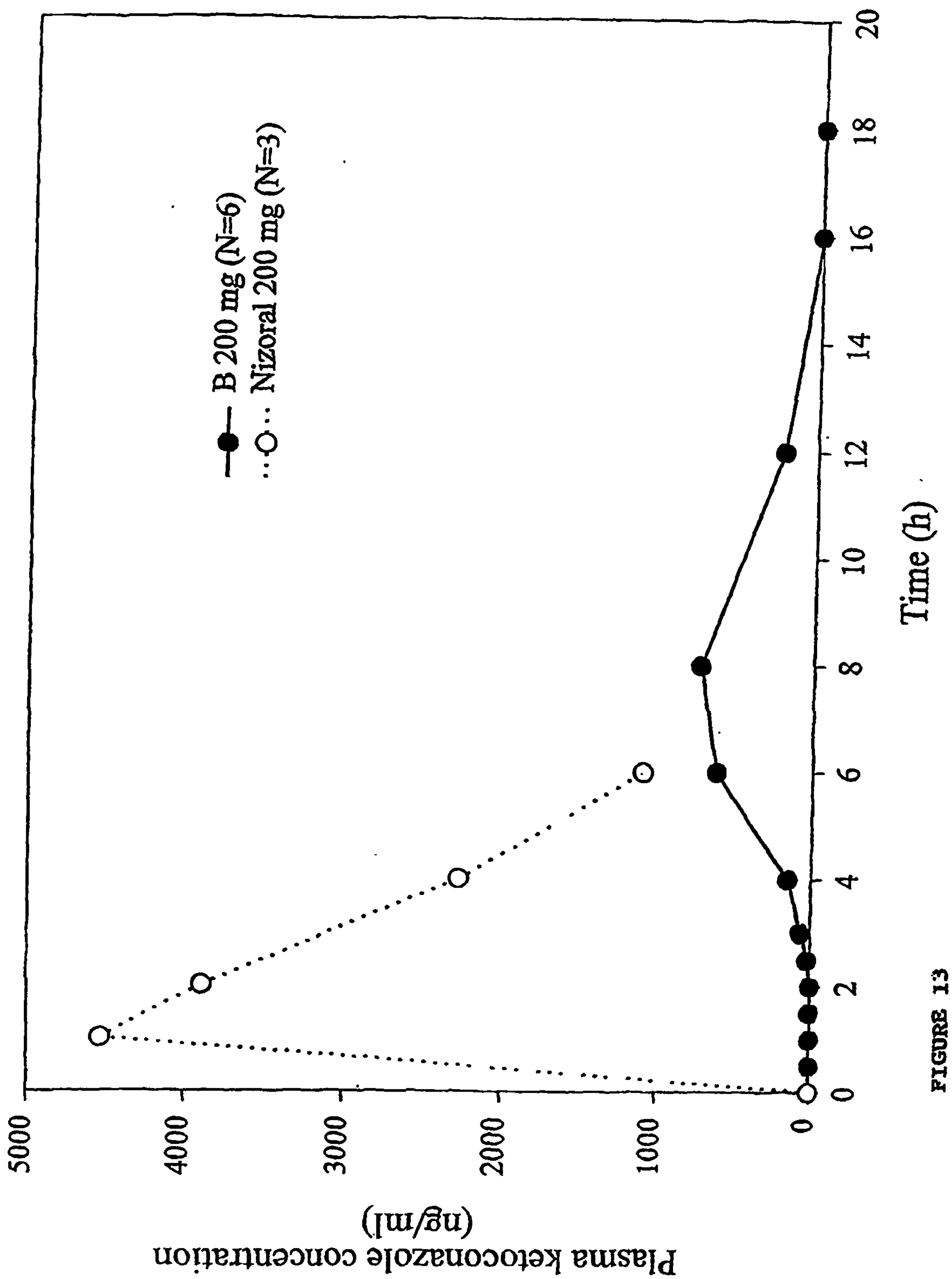
B 200 mg (N=6)
Nizoral 200 mg (N=3)
Plasma ketoconazole concentration (ng/ml)
0
1000
2000
3000
4000
5000
Time (h)
0
2
4
6
8
10
12
14
16
18
20

FIGURE 13

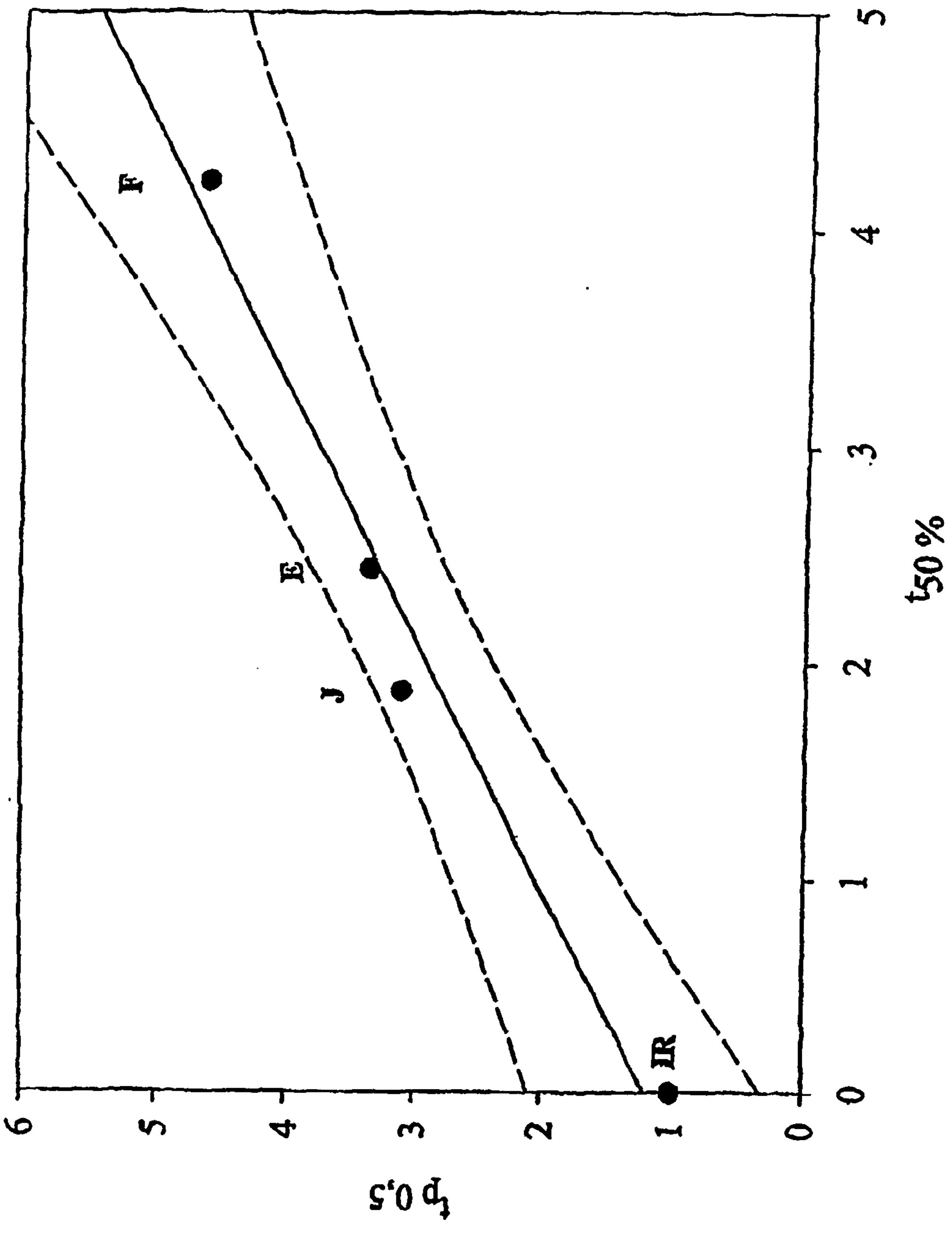


FIGURE 14

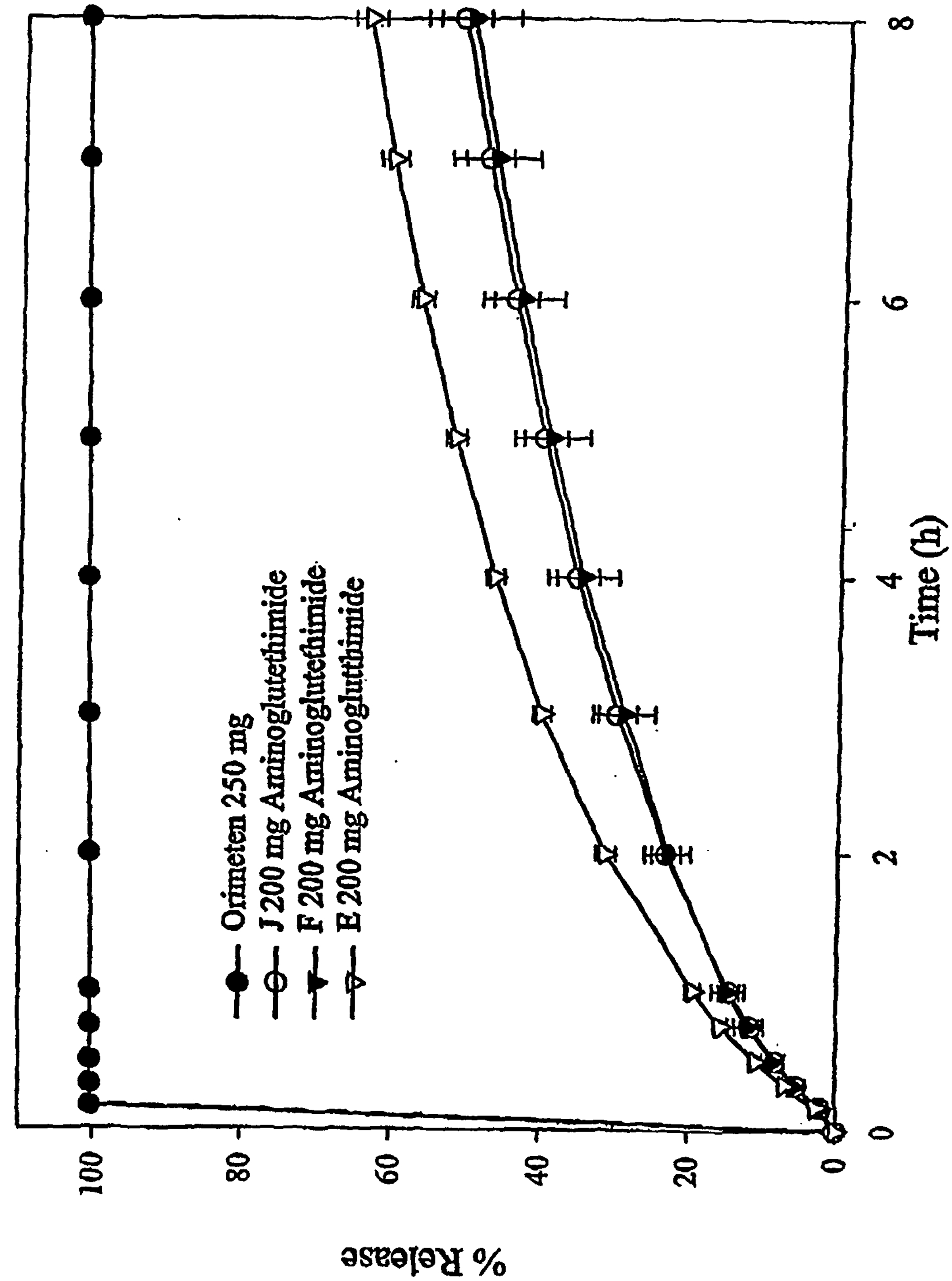
Orimeten 250 mg
J 200 mg Aminoglutethimide
F 200 mg Aminoglutethimide
E 200 mg Aminogluthimide
% Release
0
20
40
60
80
100
Time (h)
0
2
4
6
8

FIGURE 15

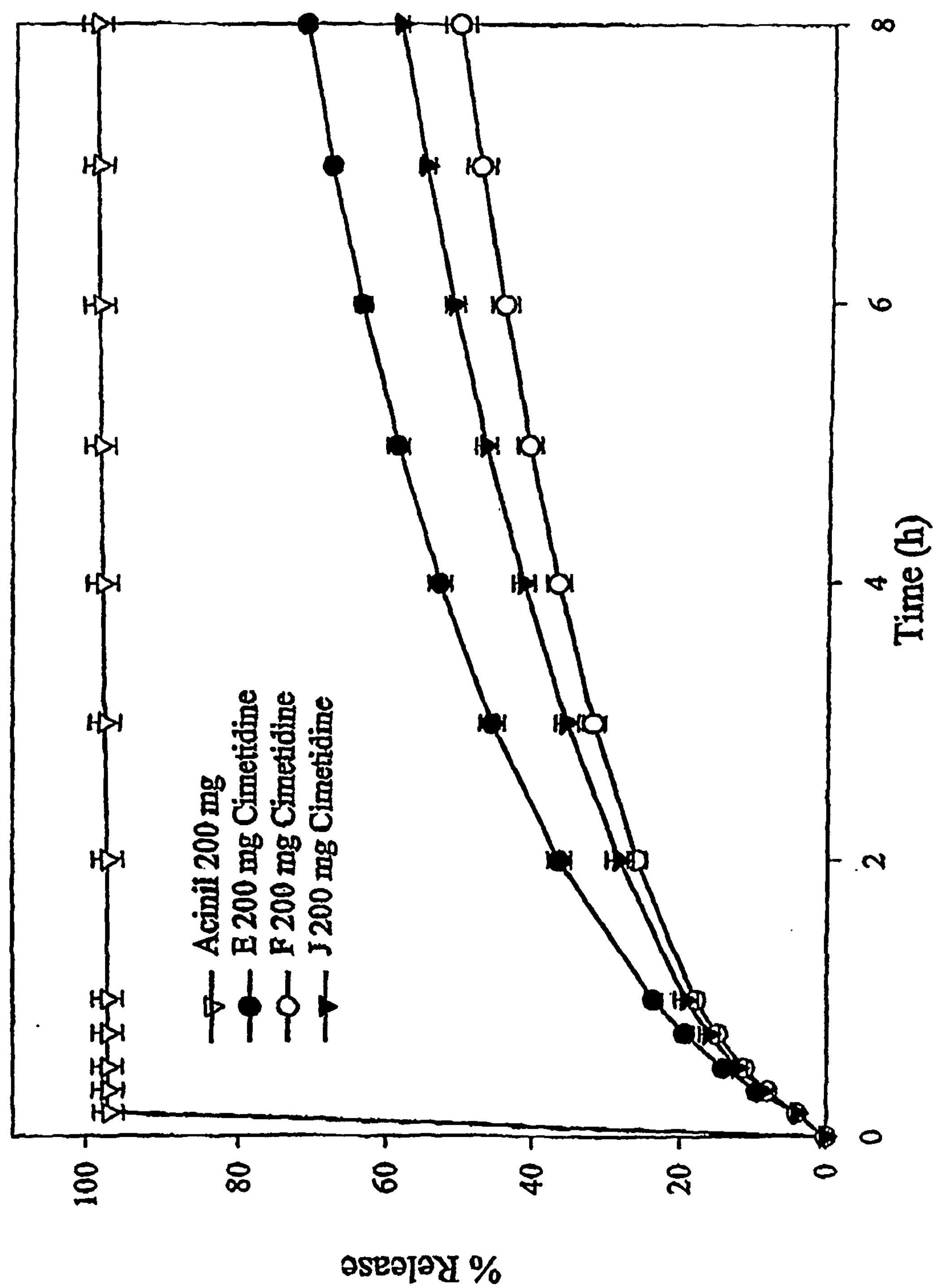

Acinil 200 mg
E 200 mg Cimetidine
F 200 mg Cimetidine
J 200 mg Cimetidine
% Release
0
20
40
60
80
100
Time (h)
0
2
4
6
8


FIGURE 16

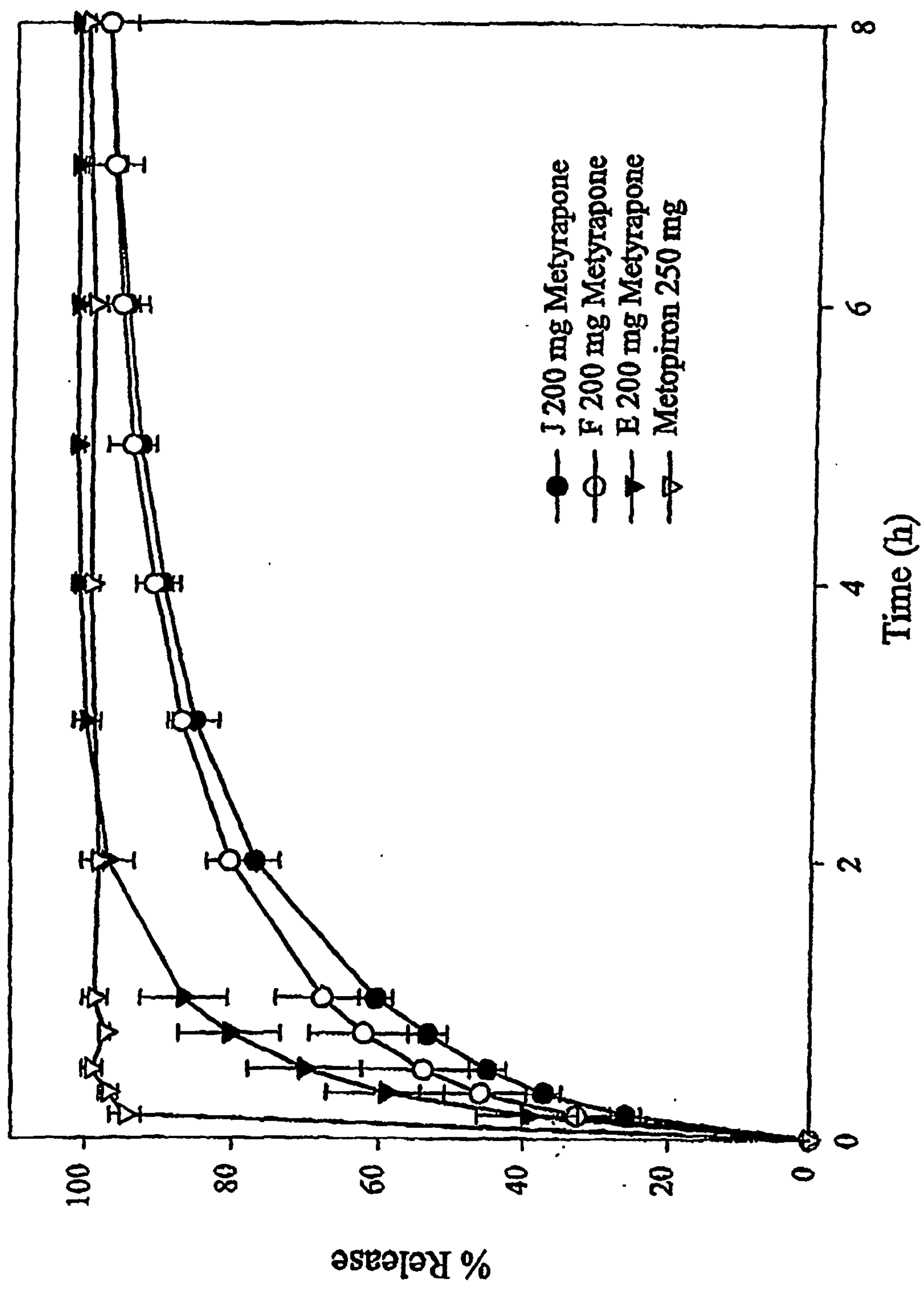
% Release
Time (h)
0
20
40
60
80
100
0
2
4
6
8
J 200 mg Metyrapone
F 200 mg Metyrapone
E 200 mg Metyrapone
Metopiron 250 mg

FIGURE 17